(19) **Europäisches Patentamt / European Patent Office / Office européen des brevets**

(11) **EP 3 027 136 B1**

(12) **EUROPÄISCHE PATENTSCHRIFT**

(45) Veröffentlichungstag und Bekanntmachung des Hinweises auf die Patenterteilung:
**08.09.2021  Patentblatt 2021/36**

(21) Anmeldenummer: **14744545.6**

(22) Anmeldetag: **28.07.2014**

(51) Int Cl.:
***A61B 46/00*** *(2016.01)*

(86) Internationale Anmeldenummer:
**PCT/EP2014/066152**

(87) Internationale Veröffentlichungsnummer:
**WO 2015/014781 (05.02.2015 Gazette 2015/05)**

(54) **WEGWERFBARE OP-ABDECKUNG MIT ÜBERFANGENER SCHWÄCHUNGSLINIE**

DISPOSABLE SURGICAL DRAPE WITH COVERED SCORE LINE

CHAMP OPÉRATOIRE À USAGE UNIQUE AVEC LIGNE AFFAIBLIE DOUBLÉE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **31.07.2013  DE 102013012744**

(43) Veröffentlichungstag der Anmeldung:
**08.06.2016  Patentblatt 2016/23**

(73) Patentinhaber: **Paul Hartmann AG**
**89522 Heidenheim (DE)**

(72) Erfinder:
- **HOFFMANN, Thomas**
  **89522 Heidenheim (DE)**
- **SCHIMEK, Gabriele**
  **73460 Hüttlingen (DE)**
- **SENGER, Tatjana**
  **89522 Heidenheim (DE)**

(56) Entgegenhaltungen:
EP-A1- 1 009 318       EP-A2- 2 151 211
WO-A1-2011/123506      JP-A- 2008 154 902
US-A- 5 109 873        US-A1- 2013 112 211

**Beschreibung**

Die vorliegende Erfindung betrifft eine wegwerfbare OP-Abdeckung umfassend ein mit einem Lochausschnitt und mit einer sich ausgehend von einem inneren und/oder einem äußeren Rand des Basistuchelements erstreckenden Schwächungslinie.

OP-Abdeckungen dienen der sterilen Abdeckung des Patienten während eines chirurgischen oder medizinischen Eingriffes. Zu den wesentlichen Eigenschaften einer OP-Abdeckung gehören die Barrierefunktion im Hinblick auf Keimdichtigkeit und Widerstand gegen Flüssigkeitspenetration, ebenso eine ausreichende Materialfestigkeit.

OP-Abdeckungen mit einem Lochausschnitt werden eingesetzt, um an einem Eingriffsbereich am Patienten eine entsprechende Barriere zu schaffen. Eingriffsbereiche am Patienten können dabei unterschiedlicher Art sein, wie beispielsweise das OP-Eingriffsfeld als solches oder auch die Bereiche bei der Durchführung von Punktionen oder zur Anbringung von Kathetern, wie Venenkatheter, oder Sonden und deren vorübergehender Verbleib am Patienten, beispielsweise zum Einbringen von Schmerzmitteln oder Betäubungsmitteln, wie bei der Durchführung einer Spinal- oder Epiduralanästhesie.

Nach der Beendigung des chirurgischen oder sonstigen medizinischen Eingriffes ist die OP-Abdeckung zu entfernen, ohne dass dabei das am Patienten angebrachte und dort zumindest vorerst zu verbleibende medizinische Hilfsmittel, wie beispielsweise eine Sonde oder ein Katheter, in dessen Stabilität und Sicherheit gefährdet wird.

Zum Entfernen von OP-Abdeckungen mit einem Lochausschnitt kann ein mechanisches Hilfsmittel, wie beispielsweise eine Schere, eingesetzt werden, um das Material der OP-Abdeckung aufzuschneiden und somit die OP-Abdeckung öffenbar und entfernbar zu machen. In nachteiliger Weise muss hierbei ein zusätzliches Hilfsmittel in den sterilen Bereich bzw. in die Nähe des sterilen Bereichs eingebracht werden, was eine Gefährdung darstellen könnte. Darüber hinaus sollte die Anwendbarkeit von OP-Abdeckungen möglichst einfach, d.h. ohne großen Aufwand und ohne viele Einzelschritte, gestaltet sein.

Für eine Erleichterung der Öffenbarkeit von OP-Abdeckungen mit Lochausschnitten ist es bekannt, bereits herstellerseitig Öffnungshilfen in oder an das Material der OP-Abdeckung einzubringen:
Zur Herstellung von öffenbaren OP-Abdeckungen wurde beispielsweise in EP 1 993 462 B1 vorgeschlagen, einen Lochausschnitt durch die beabstandeten Kanten von zwei benachbarten Materialabschnitten zu begrenzen und die außerhalb des vorgesehenen Lochausschnitts verbleibenden Kanten mittels eines Klebestreifens zu verbinden und abzudecken. Durch Abziehen dieses Klebestreifens werden die zwei Materialabschnitte wiederum vereinzelt freigesetzt und die OP-Abdeckung kann entfernt werden.

Aus dem Dokument EP 2 151 211 A1 ist eine OP-Abdeckung bekannt, welche vom Rand bis zum Lochausschnitt einen Einschnitt durch die gesamte Dicke des Materials der OP-Abdeckung aufweist, so dass zwei Kanten der OP-Abdeckung komplett voneinander getrennt sind. Über diese Schnittlinie ist ein klebender Abdeckstreifen aufgebracht, wobei im Abdeckstreifen eine partielle Schwächung für die spätere Einreißlinie bei Krafteinwirkung vorgegeben ist.

Das Dokument WO 2011/123506 A1 beschreibt eine OP-Abdeckung mit mindestens einem Lochausschnitt und mit einem durch die gesamte Dicke der ersten Materiallage hindurchgehenden Schnitt, wobei der Schnitt mit einem reißbaren Materialabschnitt überdeckt ist. Zur Erleichterung des initialen Aufreißens ist eine kantenständige Schwächungsstelle in Form einer Kerbe vorgegeben.

Die vorstehenden OP-Abdeckungen sind mit dem Nachteil verbunden, dass im Bereich der Schnittlinie im Basismaterial eine verminderte Materialfestigkeit vorliegt.

Weiter ist es bekannt, für die Öffenbarkeit von OP-Abdeckungen mit Lochausschnitten eine Schwächungslinie, wie beispielsweise eine Perforationslinie in das Basismaterial der OP-Abdeckung einzubringen. Jedoch ist damit der Verlust der Barrierefunktion, d.h. ein Verlust der Sterilität oder auch der Wasserdichtigkeit, entlang der Schwächungslinie verbunden.

Dieser Problematik entgegentretend, sind auch OP-Abdeckungen mit einem Lochausschnitt und einer vom Rand der OP-Abdeckung bis zum Lochausschnitt erstreckenden Perforationslinie bekannt, bei denen die Perforationslinie mit einem Klebestreifen überdeckt und damit abgedichtet ist. Zum Entfernen dieser OP-Abdeckung wird in einem ersten Schritt der Klebestreifen von der OP-Abdeckung abgezogen und in einem zweiten Schritt wird die unterhalb des Klebestreifens liegende Perforationslinie geöffnet. Durch diese Maßnahme lässt sich jedoch noch keine zufriedenstellende einfache Handhabung beim Öffnen und Entfernen einer OP-Abdeckung mit Lochausschnitt realisieren. Negativ hierbei ist, dass der abgezogene und damit vereinzelte Klebestreifen dann entsorgt werden muss.

Das Dokument JP 2008 154902 A offenbart eine wegwerfbare OP-Abdeckung, welche innerhalb einer Materiallage eine sich vom Rand bis zum Lochausschnitt erstreckende Schwächungslinie aufweist. Die Materiallage trennenden Bereiche der Schwächungslinie sind zumindest durch eine weitere Lage abgedeckt. Die Materiallage trennenden Bereiche zeichnen sich durch eine spezielle, so insbesondere eine spaltenweise Anordnung und/oder durch schräg einander zugewandte einzelne Trennbereiche aus, um damit die Aufreißrichtung zu optimieren und zu koordinieren. Durch Einsatz von verschiedenen Materialien, können sich innerhalb der OP-Abdeckungen ein erster und ein zweiter Bereich ergeben, durch welche sich die Schwächungslinie hindurcherstrecken kann.

Der vorliegenden Erfindung liegt die Aufgabe zugrunde, eine OP-Abdeckung mit einem Lochausschnitt bereitzustellen, welche den Anforderungen an die Barrierefunktion und der Materialfestigkeit einer OP-Abdeckung bei einer gleichzeitig einfachen Öffnungsmöglichkeit der OP-Abdeckung ohne den Einsatz von mechanischen Hilfsmitteln gerecht wird.

Diese Aufgabe wird durch eine wegwerfbare OP-Abdeckung gemäß Anspruch 1 gelöst:
Mit der vorliegenden Erfindung wurde erkannt, dass eine mit einem ersten Abdeckmaterialabschnitt in einem Lagenverbund verstärkte Schwächungslinie im Basistuchelement positiv den Anforderung an die Barrierefunktion und an die Materialfestigkeit bei einer gleichzeitig erleichterten Öffenbarkeit ohne mechanische Hilfsmittel gerecht wird.

Im Rahmen dieser Erfindung wird eine "Schwächungslinie" als eine alternierende Anordnung von ein Flachbahnmaterial trennenden Bereichen und das Flachbahnmaterial verbindenden Stegen verstanden. Dabei können die trennenden Bereiche und die verbindenden Stege (Verbindungsstege) unterschiedlich ausgebildet sein. Die das Basistuchelement trennende Bereiche können dabei beispielsweise als geradlinige, gekrümmte oder gewellte Schnitte, als Löcher, als eingestanzte und damit im Material des Basistuchelements teilweise geschwächte Abschnitte - so in Form von gequetschten Stellen ausgestaltet sein. Die Verbindungsstege können ebenso verschieden gestaltet sein, so vorzugsweise als unmittelbare und unbeschädigte Materialbrücken.

Durch die vorhandenen Verbindungsstege des Basistuchelements bleibt im Gegensatz zu einer durchgehenden Schnittlinie zumindest eine gewisse Zusammengehörigkeit des Basistuchelements erhalten, d. h. die Verbindungsstege verhindern einen unerwünschten sofortigen, unvermittelten Öffnungsvorgang. Insbesondere kann damit eine Materialfestigkeit bei Zugkräften, welche in der Ebene der Schwächungslinie auf die OP-Abdeckung bzw. auf das Basistuchelement einwirken, bereitgestellt werden.

Obschon die Verbindungsstege im Vergleich zu einer durchgehenden Schnittlinie möglicherweise einen höheren Kraftaufwand beim Öffnen einer OP-Abdeckung erfordern, tragen die bei einer Schwächungslinie vorhandenen Verbindungsstege des Basistuchelements vorteilhaft zum Öffnungsprozess bei: bei einer Krafteinwirkung in Richtung und entlang der Schwächungslinie kann im Sinne eines "geführten" Kräfteübertrags der Verbindungsstege des Basistuchelements auf den Abdeckmaterialabschnitt eine "saubere", also genauere Einreißrichtung eines Abdeckmaterialabschnitts erreicht werden. Da der erste Abdeckmaterialabschnitt sich beim Aufreißen des ersten Lagenverbundes entlang der Schwächungslinie nicht vom Basistuchelement ablöst, wird ein vereinzelt zu entsorgendes Material, wie eben ein abgelöster Abdeckmaterialabschnitt, vermieden.

Der erste Abdeckmaterialabschnitt selber weist keine Schwächungslinie auf, um damit die abdeckende und somit auch abdichtende Funktion sicher zu stellen.

Die Schwächungslinie weist eine alternierende Abfolge von Basistuchelement trennenden Bereichen mit einer Länge S1 und von Basistuchelement verbindenden Bereiche mit einer Länge S2 auf. Vorzugsweise ist die Schwächungslinie derart ausgestaltet, dass die Länge S1 der trennenden Bereiche mindestens der Länge S2 der Verbindungsstege entspricht. Weiter ist vorzugsweise die Länge S1 größer als Länge S2. Weiter vorzugsweise beträgt das Verhältnis von S1: S2 mindestens 2:1, insbesondere mindestens 4:1, weiter insbesondere mindestens 6:1, weiter insbesondere höchstens 20:1, weiter insbesondere höchstens 15:1; weiter insbesondere höchstens 12:1, weiter insbesondere höchstens 10:1. Vorzugsweise besteht die Schwächungslinie im Basistuchelement aus einer alternierenden Anordnung von trennenden, vorzugsweise geradlinigen Schnitten mit einer Länge S1 von vorzugsweise 2-6 mm, weiter bevorzugt von 3-5 mm und von Verbindungsstegen mit einer Länge S2 von 0,2 - 2,0 mm, bevorzugt von 0,3 - 1,5 mm, weiter bevorzugt von 0,4 - 1,0 mm.

Vorteilhafte Ausführungen der wegwerfbaren OP-Abdeckung können auch weitere Schwächungslinien als nur eine einzige Schwächungslinie und/oder weitere Lochausschnitte als nur einen einzigen Lochausschnitt in dem Basistuchelement aufweisen.

Vorzugsweise ist innerhalb des äußeren Randes des Basistuchelements ein weiterer zweiter Lochausschnitt angeordnet, wobei dieser zweite Lochausschnitt ebenso von einem inneren Rand begrenzt ist. Vorzugsweise ist auch dem weiteren Lochausschnitt eine weitere Schwächungslinie zugeordnet, welche sich ausgehend von dem äußeren Rand des Basistuchelements in Richtung des inneren Randes oder die sich ausgehend von dem inneren Rand in Richtung des äußeren Randes erstreckt. Vorzugsweise ist auch diese weitere Schwächungslinie von einem weiteren ersten Abdeckmaterialabschnitt überfangen und dieser weitere erste Abdeckmaterialabschnitt ist auf der ersten und/oder zweiten Oberseite des Basistuchelements unter Bildung eines weiteren ersten Lagenverbundes flächenhaft festgelegt.

In einer weiteren vorteilhaften Ausführungsform sind einem einzelnen Lochausschnitt mehr als nur eine Schwächungslinie, vorzugsweise zwei Schwächungslinien zugeordnet, wobei die Schwächungslinien in einer oder mehreren oder Kombinationen der beschriebenen Ausführungsformen der Schwächungslinie ausgestaltet sein können.

Insbesondere weist das Basistuchelement zwei, dem Lochausschnitt zugeordnete Schwächungslinien auf. Die beiden einem Lochausschnitt zugeordneten Schwächungslinien können in jeglicher Weise zueinander angeordnet sein. Insbesondere sind die zwei, einem Lochausschnitt zugeordneten Schwächungslinien in einem Winkel von 90° sich zueinander erstreckend oder in einer Linie, also in einem Winkel von 180° sich zueinander erstreckend, angeordnet.

Vorzugsweise weist der erste Lagenverbund eine Einreißkerbe an dem äußeren Rand und/oder an dem inneren Rand auf, derart, dass die Einreißkerbe in der Flucht der Schwächungslinie angeordnet ist. Der erste Lagenverbund ist ausgehend von der Einreißkerbe entlang der Schwächungslinie aufreißbar, ohne dass sich der erste Abdeckmaterialabschnitt von dem Basistuchelement löst.

Unter "Einreißkerbe" wird hier jegliche Art einer Schwächung des Lagenverbundes an dem äußeren und/oder inneren Rand verstanden. Die Einreißkerbe kann dabei beispielsweise in Form eines Schlitzes, eines Ausschnittes oder eines brechbaren geprägten Bereiches des Lagenverbundes sein. Wesentlich hierbei ist, dass die Einreißkerbe in den Lagenverbund, und dabei nur an dem äußeren und/oder inneren Rand eingebracht ist, d.h. die Erstreckung der Einreißkerbe erfolgt ausgehend vom äußeren Rand und/oder vom inneren Rand in der Flucht der anschließenden Schwächungslinie. Die Einreißkerbe weist dabei eine Länge von vorzugsweise höchstens 3 cm, weiter insbesondere von höchstens 2 cm, weiter insbesondere von höchstens 1 cm, vorzugsweise eine Länge von mindestens 0,2 cm, weiter vorzugsweise von mindestens 0,5 cm, weiter vorzugsweise von mindestens 0,75 cm auf.

Es ist vorgesehen, dass das Basistuchelement einen an den äußeren Rand angrenzenden äußeren ersten Bereich und einen an den ersten Bereich angrenzenden und sich nach innen zu dem Lochausschnitt erstreckenden inneren zweiten Bereich aufweist.

Die OP-Abdeckung kann in Abhängigkeit vom vorgesehenen Einsatzbereich eine unterschiedlich große flächenhafte Erstreckung aufweisen, wobei der OP-Abdeckung bereichsweise eine oder mehrere und auch unterschiedliche Anforderungen an die Funktionalität zugeschrieben sein können. Zum einen muss eine ausreichende Abdeckung des Patienten und/oder dessen Umfeld durch das Basistuchelement gegeben sein. Zum anderen können in Bezug auf die vorzunehmende chirurgische oder medizinische Handlung insbesondere im Bereich um den Lochausschnitt spezielle Anforderungen an die OP-Abdeckung nötig sein, wie beispielsweise Einsehbarkeit in die der Eingriffsstelle benachbarten Umgebung.

Die Differenzierung der flächenhaften Erstreckung des Basistuchelements in einen äußeren ersten Bereich und einen inneren zweiten Bereich kann auf verschiedenen Kriterien beruhen, wie beispielsweise der Ausgestaltung und/oder der Anordnung der Schwächungslinie oder/und den für das Basistuchelement bereichsweise eingesetzten Materialien und deren Eigenschaften.

Vorzugsweise unterscheiden sich der äußere erste Bereich und der innere zweite Bereich zumindest in einer Eigenschaft, mit den Eigenschaften entnommen aus der Gruppe Materialart, Lagenanzahl, Materialeigenschaften, wie Dicke, Flächengewicht, Zugfestigkeit, Dehnbarkeit, Drapierbarkeit, Reißfestigkeit, Atmungsaktivität, Absorptionskapazität, Farbe oder Transparenz.

So erweist es sich insbesondere als vorteilhaft, wenn der äußere erste Bereich aus einem besser drapierbaren Material gebildet ist als der um den Lochausschnitt angeordnete innere zweite Bereich. Insbesondere kann es vorteilhaft sein, im zweiten Bereich um den Lochausschnitt ein im Vergleich zum ersten Bereich zugfesteres Material einzusetzen, da direkt am oder um den Lochausschnitt beim Arbeiten am Patienten durchaus größere Kräfte auf die OP-Abdeckung einwirken können.

Weiter kann es vorteilhaft sein, die Atmungsaktivität, gemessen als Wasserdampfdurchlässigkeit (WVTR nach DIN 53 122-1 Ausgabe: 2001-08), des ersten Bereiches höher oder geringer auszubilden als die des zweiten Bereiches.

Weiter kann es vorteilhaft sein, die Absorptionskapazität im ersten Bereich höher oder geringer auszugestalten als im zweiten Bereich, wobei die Absorptionskapazität beispielsweise durch eine oder mehrere Lagen aus absorbierendem Material, wie insbesondere ein hydrophiles Vlies, bereitgestellt werden kann.

In einer ersten Variante der Erfindung erweist es sich als vorteilhaft, wenn sich die Schwächungslinie von dem äußeren Rand bis zu dem inneren Rand des Basistuchelements erstreckt. Die Ausführungsform einer sich vom äußeren Rand bis zum inneren Rand des Basistuchelements erstreckenden Schwächungslinie wird insbesondere vorteilhaft bei OP-Abdeckungen eingesetzt, bei denen das Basistuchelement in seiner gesamten flächenhaften Erstreckung aus der gleichen Materialart besteht.

Es ist vorgesehen, dass die Schwächungslinie sich über den ersten Bereich hinweg bis in den zweiten Bereich hinein erstreckt, wobei die Schwächungslinie in dem ersten Bereich sich von der Schwächungslinie in dem zweiten Bereich unterscheidet. Unterschiede in der Schwächungslinie sind erfindungsgemäß durch die Ausgestaltung der das Basistuchelement trennenden Bereiche und die das Basistuchelement verbindenden Bereiche gebildet.

In einer weiteren erfindungsgemäßen Variante ist vorgesehen, dass die Schwächungslinie in dem ersten Bereich angeordnet ist und dass das Basistuchelement in dem zweiten Bereich eine Schnittlinie aufweist, die in der Flucht der Schwächungslinie angeordnet ist, wobei die Schnittlinie sich in Richtung zu dem inneren Rand, vorzugsweise bis zu dem inneren Rand des Basistuchelements erstreckt.

In einer weiteren erfindungsgemäßen Variante ist vorgesehen, dass die Schwächungslinie in dem zweiten Bereich angeordnet ist und dass das Basistuchelement in dem ersten Bereich eine Schnittlinie aufweist, die in der Flucht der Schwächungslinie angeordnet ist, wobei die Schnittlinie sich in Richtung zu dem äußeren Rand, vorzugsweise bis zu dem äußeren Rand des Basistuchelements erstreckt.

In der Alternative, dass die Schwächungslinie und/oder die Schnittlinie sich in Richtung, aber nicht bis zum jeweiligen anderen inneren und/oder äußeren Rand erstreckt, ist vorteilhaft ein Steg einer Materialbrücke des Basistuchelements direkt am inneren und/oder äußeren Rand vorhanden. Eine derartige Materialbrücke hat eine Länge vorzugsweise von höchstens 8 mm, weiter vorzugsweise von höchstens 5 mm, weiter vorzugsweise von höchstens 3 mm.

Eine Schnittlinie wird im Rahmen dieser Erfindung nicht als Schwächungslinie verstanden. Die Schnittlinie wird als eine durch die gesamte Dicke des Basistuchelements hindurch reichende Trennung des Materials mit einer kontinuierlichen Erstreckung einer Länge S3 von mindestens 10 mm, weiter insbesondere von mindestens 20 mm, weiter insbesondere von mindestens 30 mm, weiter insbesondere von mindestens 40 mm verstanden.

Die Schnittlinie kann dabei auf unterschiedliche Weise in das Basistuchelement eingebracht worden sein, so beispielsweise durch ein Schneidemesser, ein Stanzwerkzeug oder durch Ultraschall. Desweiteren kann die Schnittlinie als geradliniger, gekrümmter oder gewellter Schnitt ausgestaltet sein. Vorzugsweise ist die Schnittlinie ein geradliniger Schnitt mit einer Länge S3 von mindestens 10 mm.

Eine durch die gesamte Dicke des Basistuchelements hindurchgehende Schnittlinie kann insbesondere in einem ersten oder zweiten Bereich vorteilhaft sein, wenn das in dem jeweiligen anderen zweiten oder ersten Bereich des Basistuchelements eingesetzte Material beispielsweise zugfester, reißfester oder starrer ist.

Die in das Basistuchelement zusätzlich zu der Schwächungslinie und in der Flucht zur Schwächungslinie eingebrachte Schnittlinie ist vorzugsweise überdeckt, um auch in diesen Bereichen des Basistuchelements die Barrierefunktion aufrechterhalten zu können.

Vorteilhafterweise ist dabei ein zweiter Abdeckmaterialabschnitt entlang der Schnittlinie und die Schnittlinie dabei überfangend auf der ersten und/oder zweiten Oberseite des Basistuchelements zur Bildung eines zweiten Lagenverbundes flächenhaft festgelegt und der zweite Lagenverbund ist entlang der Schnittlinie aufreißbar, ohne dass sich der zweite Abdeckmaterialabschnitt von dem Basistuchelement löst.

Weiter vorzugsweise kann in der Ausführungsform mit mehreren Schwächungslinien in der Flucht aller Schwächungslinien jeweils eine weitere Schnittlinie angeordnet sein, wobei auch diese jeweils weitere Schnittlinie von einem weiteren zweiten Abdeckmaterialabschnitt überfangen ist und der zweite Abdeckmaterialabschnitt dabei flächenhaft festgelegt ist, und zwar analog wie vorangehend beschrieben, unter Ausbildung eines weiteren zweiten Lagenverbundes.

Insbesondere weist auch der zweite Abdeckmaterialabschnitt keine Schwächungslinie auf.

Zum Überfangen der Schwächungslinie und der in der Flucht dazu angeordneten Schnittlinie sind der erste und zweite Abdeckmaterialabschnitt vorzugsweise aus einem einzigen Materialabschnitt gebildet.

In einer Variante kann auch vorgesehen sein, dass der erste Abdeckmaterialabschnitt auf einer der beiden Oberseiten, also der ersten oder der zweiten Oberseite, und der zweite Abdeckmaterialabschnitt auf der anderen der beiden Oberseiten auf dem Basistuchelement festgelegt sind.

Die flächenhafte Festlegung des ersten und/oder zweiten Abdeckmaterialabschnitts auf dem Basistuchelements erfolgt mittels Fügemittel, vorzugsweise mittels Fügemittel entnommen aus der Gruppe Klebemittel, wie insbesondere doppelseitiges Klebeband, Kleber, insbesondere eine Haftkleberbeschichtung, Schweißstellen, wie insbesondere Ultraschallschweißstellen, thermische Schweißstellen oder Kalanderschweißstellen, oder Thermolaminierung oder Thermobonding.

Für die flächenhafte Festlegung des ersten und/oder zweiten Abdeckmaterialabschnitts auf die Oberseite des Basistuchelements wird vorteilhafterweise ein Klebemittel mit einer hohen Klebkraft eingesetzt, wie vorzugsweise ein Acrylatkleber eingesetzt. Für ein Klebemittel wird weiter vorzugsweise eine Haftkleberbeschichtung gewählt. Insbesondere vorteilhaft weist der erste und/oder zweite Abdeckmaterialabschnitt eine einseitige Haftkleberbeschichtung, insbesondere aus Acrylatkleber auf.

Insbesondere weist der eingesetzte Kleber, insbesondere die Haftkleberbeschichtung, ein Flächengewicht von mindestens 20 g/m$^2$, weiter insbesondere von mindestens 25 g/m$^2$, weiter insbesondere von mindestens 30 g/m$^2$, weiter insbesondere von höchstens 70 g/m$^2$, weiter insbesondere von höchstens 60 g/m$^2$, weiter insbesondere von höchstens 50 g/m$^2$, weiter insbesondere von höchstens 40 g/m$^2$ auf.

Insbesondere weist der Abdeckmaterialabschnitt mit einer einseitigen Haftkleberbeschichtung eine Haftkraft bei 180° gegen eine Polyethylen-Oberfläche, vermessen nach der beschriebenen Methode, von mindestens 2N/25mm, weiter vorzugsweise von mindestens 3N/25mm, weiter vorzugsweise von mindestens 4N/25mm auf.

Mit einer hohen Haftkraft des Klebemittels wird ein stabiler adhäsiver flächenhafter Lagenverbund zwischen Abdeckmaterialabschnitt und Basistuchelement geschaffen, so dass beim Einreißen des Lagenverbundes entlang der Schwächungslinie und/oder der Schnittlinie sich der Abdeckmaterialabschnitt nicht vom Basistuchelement löst.

Der zum Überfangen der Schwächungslinie und/oder Schnittlinie eingesetzte erste und/oder zweite Abdeckmaterialabschnitt besteht insbesondere vorteilhaft aus einem polymeren Material, insbesondere aus polymerem Material entnommen aus der Gruppe Polyethylen, Polypropylen, Polyurethan oder Mischungen davon.

Ein aus polymerem Material bestehender Abdeckmaterialabschnitt kann aufgrund der den polymeren Materialien zumeist inhärent hydrophoben Eigenschaft vorteilhaft zur Verstärkung der Barrierefunktion beitragen.

Insbesondere können im Falle eines ersten und zweiten Abdeckmaterialabschnittes der erste und der zweite Abdeckmaterialabschnitt aus dem gleichen polymeren Material bestehen.

Der Abdeckmaterialabschnitt ist vorzugsweise als Folie (synonym auch als Film bezeichnet) ausgebildet. Die für den Abdeckmaterialabschnitt vorzugsweise eingesetzte polymere Folie weist vorzugsweise geringe Rückstellkräfte auf, d.h. die Folie zeigt im Zugversuch kaum die Tendenz, wieder in die ursprüngliche Länge zurückzukehren. Vorzugsweise weist die polymere Folie des Abdeckmaterialabschnittes eine Reißkraft von mindestens 15N/25mm, weiter vorzugsweise von mindestens 20N/25mm, weiter vorzugsweise von höchstens 40N/25mm, weiter vorzugsweise von höchstens 35N/25mm, weiter vorzugsweise von höchstens 30N/25mm, gemessen nach der beschriebenen Methode, auf. Die Reißkraft beschreibt die maximale Kraft, also die erste maximale Kraftspitze im Kräfteverlauf der Messung, welche benötigt wird, um einen definierten Prüfling zu zerreißen.

Vorzugsweise weist die polymere Folie des Abdeckmaterialabschnittes eine Reißdehnung ausgedrückt in % von mindestens 400%, weiter insbesondere von mindestens 450%, weiter insbesondere von 500%, weiter insbesondere von mindestens 550%, weiter insbesondere von mindestens 600%, weiter insbesondere von mindestens 650%, weiter insbesondere von höchstens 900%, weiter insbesondere von höchstens 850%, weiter insbesondere von höchstens 800%, weiter insbesondere von höchstens 750%, weiter insbesondere von höchstens 700%, weiter insbesondere von höchstens 650% gemessen nach der beschriebenen Methode, auf. Die Reißdehnung wird als die am Messpunkt der Reißkraft maximal erreichte Dehnung (ausgedrückt in %) verstanden.

Eine hohe Reißdehnung der Folie des Abdeckmaterialabschnittes unterstützt vorteilhaft die Materialfestigkeit der OP-Abdeckung bei Krafteinwirkungen in der Ebene bzw. parallel zu der Ebene des Abdeckmaterialabschnitts, da es zu einer Verzögerung des Reißens kommt. Andererseits sind ein zu großer freier und ungebundener Bereich der Folie und damit ein großer flächenhafter Spielraum eines Dehnungsbereiches zu vermeiden, um ein erleichtertes Aufreißen des Lagenverbundes entlang der Schwächungslinie bzw. der Schnittlinie zu ermöglichen. Es wurde erkannt, dass die Dehnbarkeit der Folie des Abdeckmaterialabschnittes durch die flächenhafte Festlegung des Abdeckmaterialabschnittes an eine Oberseite des Basistuchelements begrenzt wird, da eine Dehnung nur noch in den nicht an das Basistuchelement gebundenen Bereichen, also nur in den trennenden Bereichen der Schwächungslinie bzw. der Schnittlinie und damit ein kontrolliertes Trennen möglich ist.

Vorzugsweise weist die Folie des Abdeckmaterialabschnittes ein Flächengewicht von 20 - 50 g/m$^2$, weiter insbesondere von 25 - 45 g/m$^2$, weiter insbesondere von 30 - 40 g/m$^2$ auf.

Weiter vorteilhaft ist der Abdeckmaterialabschnitt streifenförmig. Insbesondere erstreckt sich der Abdeckmaterialabschnitt beidseits der Schwächungslinie oder der Schnittlinie über eine Querstreckung von je mindestens 10 mm, weiter insbesondere von 15 mm, weiter insbesondere von 20 mm, weiter insbesondere von höchstens 50mm, weiter insbesondere von höchstens 40 mm.

Insbesondere kann der erste und/oder zweite Abdeckmaterialabschnitt oder jeder weitere Abdeckmaterialabschnitt aus einem polymeren Flachmaterial gebildet sein, wobei eine Oberseite des Flachmaterials eine Haftkleberbeschichtung aufweist. Insbesondere vorteilhaft ist der Abdeckmaterialabschnitt als ein Polyurethanfilm mit einem einseitig aufgebrachten Polyacrylatkleber ausgebildet.

In besonders vorteilhafter Weise weist das Basistuchelement ein Material auf oder ist aus dem Material gebildet, welches ausgewählt ist aus der Gruppe Folienmaterial, ein- oder mehrlagiger Vliesstoff, insbesondere Laminat aus einer oder mehreren Spinnvlies- und/oder Meltblownvlieslagen, Vlies-Folien-Verbund, insbesondere Vlies-Folien-Verbund mit einem hydrophilem Vliesstoff mit ein oder mehreren Lagen aus Spunbond und/oder Meltblown.

Das im Basistuchelement eingesetzte Vliesmaterial, insbesondere das Vliesmaterial im Vlies-Folien-Verbund und/oder das Vliesmaterial auf einer Oberseite des Basistuchelements besteht vorzugsweise aus einer oder mehreren Spinnvlies- und/oder Meltblownvlieslagen aus polymeren Materialien, insbesondere ausgewählt aus der Gruppe Polyethylen, Polypropylen oder Mischungen, insbesondere ein hydrophiles PP-Spinnvlies.

Das im Basistuchelement eingesetzte Vliesmaterial, insbesondere das Vliesmaterial im Vlies-Folien-Verbund und/oder das Vliesmaterial auf insbesondere der zweiten Oberseite des Basistuchelements weist ein Flächengewicht von vorzugsweise 10 - 40 g/m$^2$, insbesondere 10-35 g/m$^2$, weiter insbesondere 15-35 g/m$^2$, weiter insbesondere 20-35 g/m$^2$, weiter insbesondere 25 - 35 g/m$^2$ auf.

Die Laminierung des Vlies-Folien-Verbundes kann auf jegliche Art erfolgen. Vorzugsweise sind Vlies und Folie mittels Thermolaminierung (d.h. das Folienmaterial wird dabei im hergestellten warmen Zustand direkt an das Vlies gebunden), mittels Thermobonding (d.h. das bereits erkaltete Folienmaterial wird unter erneuter Wärmeeinwirkung an das Vlies gebunden) und/oder mittels Kleber, insbesondere Hotmeltkleber verbunden.

Das im Basistuchelement eingesetzte Folienmaterial, insbesondere das Folienmaterial der ersten Oberseite des Basistuchelements und/oder insbesondere die Folie im Vlies-Folien-Verbund ist vorzugsweise eine nicht mikroporöse Folie aus polymeren Materialien ausgewählt aus der Gruppe Polyethylen, Polypropylen, Polyurethan oder Mischungen davon, insbesondere ist es eine nicht poröse Polyethylenfolie.

Das im Basistuchelement eingesetzte Folienmaterial, insbesondere das Folienmaterial der ersten Oberseite des Basistuchelements und/oder die Folie im Vlies-Folien-Verbund des Basistuchelements weist ein Flächengewicht von vorzugsweise 15-40 g/m$^2$, insbesondere 20 - 35 g/m$^2$, weiter insbesondere von 20-30 g/m$^2$ auf.

Vorzugsweise weist das im zweiten Bereich angeordnete Folienmaterial ein größeres Flächengewicht auf als das im ersten Bereich vorhandene Folienmaterial. Vorzugsweise weist das Folienmaterial im zweiten Bereich eine Flächengewicht von mindestens 30 g/m$^2$, insbesondere von mindestens 40 g/m$^2$, weiter insbesondere von mindestens 50 g/m$^2$, weiter insbesondere von mindestens 60 g/m$^2$, weiter insbesondere von höchstens 80 g/m$^2$, weiter insbesondere von höchstens 70 g/m$^2$ auf.

Die jeweilige erste Oberseite oder zweite Oberseite des Basistuchelements und damit auch die Sichtseite der OP-Abdeckung können im Anwendungszustand der OP-Abdeckung sowohl der patientenzugewandten oder der patientenabgewandten Seite entsprechen. Die Anordnung der OP-Abdeckung und damit des Basistuchelements am Patienten obliegt dem medizinischen Personal, auch verbunden mit dem Anforderungsprofil für den jeweiligen Einsatzbereich der OP-Abdeckung. Vorzugsweise ist die erste Oberseite in der Anwendung der OP-Abdeckung als die patientenzugewandte Oberseite und die zweite Oberseite als die patientenabgewandte Oberseite festgelegt.

Die Ausgestaltung derjenigen Oberseite des Basistuchelements, welche innerhalb des ersten und/oder zweiten Lagenverbundes dem Abdeckmaterialabschnitt für deren flächenhafte Festlegung zugewandt ist, kann vorteilhaft ausgewählt werden.

So ist es insbesondere vorteilhaft, wenn zumindest die innerhalb des ersten und/oder des zweiten Lagenverbundes dem Abdeckmaterialabschnitt zugewandte Oberseite des Basistuchelements ein Folienmaterial aufweist. Vorteilhafterweise ist der erste und/oder zweite Lagenverbund auf der ersten Oberseite des Basistuchelements angeordnet, so dass in dem Bereich des Lagenverbundes diese erste Oberseite ein Folienmaterial aufweist. Insbesondere vorteilhaft ist die gesamte erste Oberseite aus einem Folienmaterial gebildet. Vorzugsweise entspricht in dieser Ausgestaltung die erste Oberseite in der Anwendung der OP-Abdeckung der dem Patienten zugewandten Oberseite.

Es wurde erkannt, dass die flächenhafte Festlegung des Abdeckmaterialabschnitts auf einer ein Folienmaterial aufweisenden oder auf einer aus Folienmaterial gebildeten Oberseite vorteilhaft ist. Es können damit insbesondere Feuchtigkeitsunterwanderungen unterbunden werden, welche sich ansonsten vor allem in Bereichen auf Oberflächen mit einer faserigen Textur und/oder mit absorbierenden Eigenschaften, wie bspw. bei Vliesmaterialien, insbesondere hydrophilen Vliesmaterialien, ergeben können. Diese Ausgestaltung trägt insbesondere vorteilhaft zur Gewährleistung der Barrierefunktion einer OP-Abdeckung bei.

Die Oberseiten des Basistuchelements können gleich oder verschieden sein. Vorteilhafterweise unterscheiden sich die erste und zweite Oberseite des Basistuchelements voneinander.

So ist in einer vorteilhaften Ausbildung der OP-Abdeckung die erste Oberseite aus einem anderen Material oder einem anderen Materialverbund gebildet ist als die zweite Oberseite, wobei eine der Oberseiten, insbesondere die zweite Oberseite des Basistuchelements ein absorbierendes Material, insbesondere ein Vliesmaterial, insbesondere ein hydrophiles Vliesmaterial aufweist oder daraus gebildet ist. Vorzugsweise entspricht in dieser Ausgestaltung die zweite Oberseite in der Anwendung der OP-Abdeckung der patientenabgewandten Oberseite. Somit steht die zweite Oberseite vorteilhaft zum Aufsaugen für die durch den chirurgischen oder medizinischen Eingriff bedingten Flüssigkeitsaustritte zur Verfügung.

In einer weiteren vorteilhaften Ausführung weist der erste Bereich des Basistuchelements ein Vlies-Folien-Laminat auf, insbesondere ist der erste Bereich aus einem Vlies-Folien-Laminat gebildet und der zweite Bereich weist eine insbesondere transparente Folie auf, insbesondere ist der zweite Bereich aus einer insbesondere transparenten Folie gebildet.

Der Einsatz eines Vlies-Folien-Laminates im ersten Bereich des Basistuchelements ermöglicht ein Basistuchelement mit zwei verschiedenen Oberseiten, so dass diese Ausführungsform der OP-Abdeckung anwendungsspezifisch eingesetzt werden kann.

Ein Folienmaterial im zweiten Bereich, also in dem sich um den Lochausschnitt erstreckenden Bereich, schafft vorteilhaft eine Dichtigkeit gegen Flüssigkeitsdurchtritt. Die mögliche Transparenz verschafft der behandelnden Person eine Einsehbarkeit in die am Eingriffsbereich im Lochausschnitt direkt benachbarte Umgebung.

Insbesondere bevorzugt ist im ersten Bereich des Basistuchelements ein zweischichtiger Materialverbund in Form eines Vlies-Folien-Laminats bestehend aus einer flüssigkeitsundurchlässigen porenfreien PE-Folie, insbesondere mit einem Flächengewicht von 20-30 g/m$^2$ und einem hydrophilen PP-Spinnvlies, insbesondere mit einem Flächengewicht von 25 - 35 g/m$^2$ eingesetzt. Im zweiten Bereich des Basistuchelements ist insbesondere eine porenfreie PE-Folie, insbesondere mit einem Flächengewicht von 30-80g/m$^2$ eingesetzt.

Der erste Abdeckmaterialabschnitt wird in seiner Funktion der Abdeckung und des Überfangens der Schwächungslinie und der zweite Abdeckmaterialabschnitt wird in seiner Funktion der Abdeckung und des Überfangens der Schnittlinie zugeordnet.

Es erweist sich weiter als vorteilhaft, wenn ein weiterer erster und/oder zweiter Abdeckmaterialabschnitt auf einer Oberseite des Basistuchelements entlang der Schwächungslinie und/oder der Schnittlinie und die Schwächungslinie und/oder die Schnittlinie dabei überfangend festgelegt ist, derart, dass die Schwächungslinie und/oder die Schnittlinie sowohl auf der ersten als auch auf der zweiten Oberseite von einem Abdeckmaterialabschnitt überfangen ist.

Das Festlegen eines weiteren ersten und/oder zweiten Abdeckmaterialabschnitts kann insbesondere im Falle einer Ausgestaltung der anderen Oberseite, insbesondere der zweiten Oberseite mit einer faserhaltigen Struktur/Textur vorteilhaft sein, um somit die beim Aufreißen des Lagenverbundes auf der anderen bzw. der zweiten Oberseite möglicherweise entstehenden Partikel, wie Faserbruchstücke, damit abzufangen und somit als Kontaminationsherde des sterilen Bereichs zu reduzieren bzw. zu unterbinden. Der weitere erste und/oder zweite Abdeckmaterialabschnitt kann insbesondere vorteilhaft wie der auf der anderen, insbesondere auf der ersten Oberseite festgelegte erste und / oder zweite Abdeckmaterialabschnitt ausgestaltet sein. Alternativ kann vorteilhaft auch vorgesehen sein, dass sich die auf der ersten und zweiten Oberseite festgelegten ersten und/oder zweiten Abdeckmaterialabschnitte voneinander unterscheiden, wie beispielsweise in der Materialzusammensetzung oder den Materialeigenschaften.

In der Ausgestaltung, dass die Schwächungslinie sowohl auf der ersten als auch auf der zweiten Oberseite von einem ersten Abdeckmaterialabschnitt überfangen ist und der jeweilige erste Abdeckmaterialabschnitt auf dem Basistuchelement flächenhaft festgelegt ist, umfasst der jeweilige erste Lagenverbund somit die Abfolge erster Abdeckmaterialabschnitt, Basistuchelement und erster Abdeckmaterialabschnitt und die dazwischen angeordneten Fügemittel. Analog umfasst der zweite Lagenverbund den auf der einen Oberseite festgelegten zweiten Abdeckmaterialabschnitt und den auf der anderen Oberseite flächenhaft festgelegten und die Schnittlinie überfangenden zweiten Abdeckmaterialabschnitt, also somit die Abfolge zweiter Abdeckmaterialabschnitt, Basistuchelement und zweiter Abdeckmaterialabschnitt und die dazwischen angeordneten Fügemittel.

In einer Variante kann auch vorgesehen sein, dass der erste Abdeckmaterialabschnitt auf einer der beiden Oberseiten, also der ersten oder der zweiten Oberseite, und der zweite Abdeckmaterialabschnitt auf der anderen der beiden Oberseiten auf dem Basistuchelement festgelegt sind.

Im Anwendungszustand einer OP-Abdeckung können unterschiedliche Kräfte auf die OP-Abdeckung, insbesondere auf die Schwächungslinie und/oder die Schnittlinie des Basistuchelements und den jeweiligen Lagenverbund aus Abdeckmaterialabschnitt und Basistuchelement einwirken. Der Lagenverbund ist dabei derart ausgebildet, dass zum einen ein Aufreißen und damit ein vom Anwender gewolltes, bewusst gesteuertes Öffnen des Lagenverbundes und damit des Basistuchelements bzw. der OP-Abdeckung möglich sind. Zum anderen ist der Bereich des Lagenverbundes derart ausgebildet, dass ein ungewolltes Öffnen erschwert oder vermieden wird.

Zum Aufreißen des Lagenverbundes ist eine Zugkraft entlang dem Verlauf der Schwächungslinie und/oder Schnittlinie denkbar, wobei die Zugkraft senkrecht zu der Ebene des Basistuchelements und des Abdeckmaterialabschnitt einwirkt: Diese Art der Krafteinwirkung kann durch entgegengesetzte Zugkräfte in einem Winkel von 180° an den beidseits der Schwächungslinie bzw. Schnittlinie vorhandenen und sich ergebenen Schenkel des Basistuchelements durch eine Vertikalbewegung jeweils nach oben und nach unten ausgeübt sein. Diese Form der Krafteinwirkung wird als die sogenannte Weiterreißkraft verstanden. Die Weiterreißkraft stellt eine Möglichkeit der aktiv zugeführten Krafteinwirkung zum Aufreißen des Lagenverbundes dar. Die Methode zur Messung der mittleren Weiterreißkraft ist nachfolgend im Detail beschrieben.

In einer vorteilhaften Ausbildung der Erfindung ist der erste Lagenverbund entlang der Schwächungslinie mit einer mittleren Weiterreißkraft von mindestens 5N, insbesondere von mindestens 7N, weiter insbesondere von mindestens 9N, weiter insbesondere von höchstens 15N, weiter insbesondere von höchstens 13N, weiter insbesondere von höchstens 11N, gemessen nach der beschriebenen Methode, aufreißbar.

In einer weiteren vorteilhaften Ausbildung der Erfindung ist der zweite Lagenverbund entlang der Schnittlinie mit einer mittleren Weiterreißkraft von mindestens 2N, insbesondere von mindestens 3N, weiter insbesondere von mindestens 4N, weiter insbesondere von höchstens 8N, weiter insbesondere von höchstens 7N, weiter insbesondere von höchstens 6N gemessen nach der beschriebenen Methode aufreißbar.

Um im Gebrauchs- und Anwendungszustand der OP-Abdeckung, so z.B. beim Drapieren der OP-Abdeckung, ein unerwünschtes und unvorhergesehenes Öffnen zu vermeiden, ist eine Materialfestigkeit der OP-Abdeckung auch innerhalb der Ebene der Schwächungslinie bzw. parallel zu dieser Ebene wünschenswert. Dazu tragen u.a. die Verbindungsstege der Schwächungslinie bei. Für die Charakterisierung der Festigkeit der OP-Abdeckung, bedingt durch die Verbindungsstege des Basistuchelements und/oder dem darüber angebrachten Abdeckmaterialabschnitt kann die sogenannte Zugfestigkeit herangezogen werden. Die Zugkraftfestigkeit wird durch die Kraft beschrieben, welche benötigt wird, um im ersten Lagenverbund im Bereich der Schwächungslinie eine Trennung in zwei Hälften zu bewirken. Die Zugkraftfestigkeit kann auch die Kraft beschreiben, welche benötigt wird, um die Trennung des Lagenverbundes beidseits der Schnittlinie zu bewirken. Die Methode zur Messung der Zugfestigkeit ist nachfolgend im Detail beschrieben.

In einer vorteilhaften Ausführung weist der erste Lagenverbund eine Zugfestigkeit von mindestens 15 N/50mm, insbesondere von mindestens 25 N/50mm, weiter insbesondere von mindestens 35 N/50mm, weiter insbesondere von mindestens 40N/50mm, weiter insbesondere von höchstens 75 N/50mm, weiter insbesondere von höchstens 65 N/50mm, weiter insbesondere von höchstens 55N/50mm, weiter insbesondere von höchstens 50N/50mm, gemessen nach der beschriebenen Methode auf.

In einer weiteren vorteilhaften Ausführung weist der zweite Lagenverbund eine Zugfestigkeit von mindestens 15 N/50mm, insbesondere von mindestens 25 N/50mm, weiter insbesondere von mindestens 35 N/50mm, weiter insbesondere von mindestens 40N/50mm, weiter insbesondere von höchstens 75 N/50mm, weiter insbesondere von höchstens 65 N/50mm, weiter insbesondere von höchstens 55N/50mm, weiter insbesondere von höchstens 50N/50mm, gemessen nach der beschriebenen Methode auf. Beim Aufreißen des Lagenverbundes löst sich erfindungsgemäß der Abdeckmaterialabschnitt nicht vom Basistuchelement, d.h. der flächenhafte Lagenverbund zwischen Abdeckmaterialabschnitt und Basistuchelement bleibt bestehen. Der flächenhafte Lagenverbund weist damit einen Delaminierungswiderstand auf. Der Delaminierungswiderstand wird als die Kraft ausdrückt, welche zum Trennen des Lagenverbunds von Abdeckmaterial und Basistuchelement notwendig ist.

In einer vorteilhaften Ausführung weist der erste und/oder der zweite Lagenverbund einen mittleren Delaminierungswiderstand von mindestens 1,0 N/50mm, insbesondere von mindestens 2,0 N/50mm, weiter insbesondere von mindestens 3,0 N/50mm, gemessen nach der beschriebenen Methode, auf.

Vorzugsweise weist der erste Lagenverbund entlang der Schwächungslinie und/oder der zweite Lagenverbund entlang der Schnittlinie einen Durchdringungswiderstand von mindestens 10 mbar, weiter insbesondere von mindestens 30 mbar, weiter insbesondere von mindestens 50 mbar, weiter insbesondere von mindestens 75 mbar, weiter insbesondere von mindestens 100 mbar, gemessen nach der beschriebenen Methode, auf.

Weitere Merkmale, Vorteile und Einzelheiten der Erfindung ergeben sich aus den beigefügten Patentansprüchen und aus der zeichnerischen Darstellung und nachfolgenden Beschreibung bevorzugter Ausführungsformen der Erfindung.

Die Erfindung soll im Folgenden anhand von Figuren näher erläutert werden, dabei zeigen

Figur 1: schematisch eine erfindungsmäße OP-Abdeckung mit einer Schwächungslinie,

Figur 2: schematisch eine weitere Ausführungsform einer erfindungsgemäßen OP-Abdeckung,

Figur 3: schematisch eine weitere Ausführungsform einer erfindungsgemäßen OP-Abdeckung,

Figur 4: schematisch einen ersten Lagenverbund in der Schnittansicht I - I von Figur 1,

Figur 5: schematisch einen zweiten Lagenverbund in der Schnittansicht II - II von Figur 2,

Figur 6: schematisch einen Ausschnitt eines entlang der Schwächungslinie aufgerissenen Lagenverbundes,

Figur 7: schematisch eine Schnittansicht eines Basistuchelements mit auf beiden Oberseiten des Basistuchelements festgelegtem und dabei die Schwächungslinie überfangenden Abdeckmaterialabschnitt,

Figur 8: schematisch eine Schnittansicht eines Basistuchelements mit auf beiden Oberseiten des Basistuchelements festgelegtem und dabei die Schnittlinie überfangenden Abdeckmaterialabschnitt,

Figuren 9, 10: schematisch jeweils eine weitere erfindungsgemäße OP-Abdeckung mit zwei Schwächungslinien,

Figur 11: schematisch eine weitere erfindungsgemäße OP-Abdeckung mit zwei Lochausschnitten.

Figuren 12a, 12b: schematisch die Versuchsanordnung für die Messung der mittleren Weiterreißkraft, und

Figur 13: schematisch die Versuchsanordnung für die Messung der Zugfestigkeit,

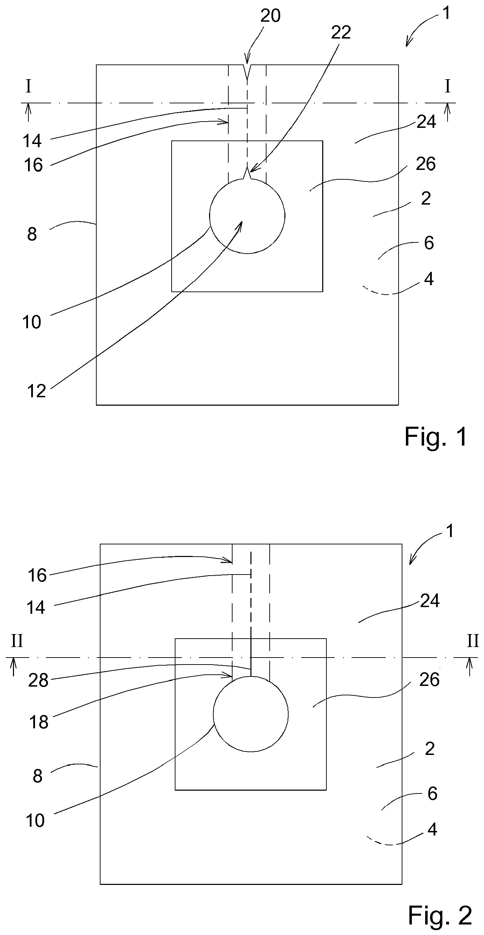

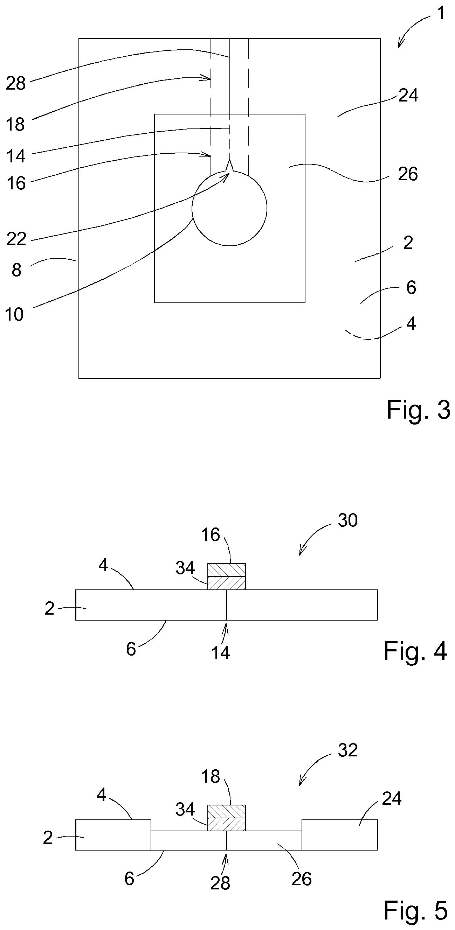

Die Figur 1 zeigt schematisch eine bevorzugte wegwerfbare OP-Abdeckung 1 umfassend ein flüssigkeitsundurchlässiges Basistuchelement 2 mit einer ersten Oberseite 4 und einer zweiten Oberseite 6, wobei Figur 1 die Aufsicht auf die zweite Oberseite 6 wiedergibt. Das Basistuchelement 2 erstreckt sich zwischen dem äußeren Rand 8 und dem inneren Rand 10, wobei der innere Rand 10 den Lochausschnitt 12 begrenzt. Das Basistuchelement weist einen an den äußeren Rand 8 angrenzenden äußeren ersten Bereich 24 und einen an den ersten Bereich 24 angrenzenden und sich nach innen zu dem Lochausschnitt 12 erstreckenden inneren zweiten Bereich 26 auf. Der erste Bereich 24 unterscheidet sich vom zweiten Bereich 26. So ist der zweite Bereich 26 aus einem transparenten Folienmaterial, insbesondere einer Polyethylenfolie mit einem Flächengewicht von 60-70 g/m$^2$ gebildet. Im ersten Bereich 24 des Basistuchelements 2 ist ein zweischichtiger Materialverbund, nämlich ein Vlies-Folien-Laminat eingesetzt, bestehend aus einer flüssigkeitsundurchlässigen Folienlage und einer absorbierenden Vlieslage. Das Vliesmaterial ist hierbei vorzugsweise ein hydrophiles PP-Spinnvlies, insbesondere mit einem Flächengewicht von 25 - 35 g/m$^2$. Die Folienlage ist vorzugsweise eine porenfreie Polyethylen-Folie mit einem Flächengewicht von 20-30 g/ m$^2$. Der Materialverbund des Vliesmaterials und des Folienmaterials basiert vorzugsweise auf einer thermischen Bindung. Mit dem in dem ersten und dem zweiten Bereich 24, 26 eingesetzten unterscheidbaren Material, nämlich eines Vlies-Folien-Laminates bzw. einer transparenten Folie unterscheiden sich auch die Oberseiten 4, 6 des Basistuchelements. In der Anwendung der OP-Abdeckung soll die erste Oberseite 4 die patientenzugewandte und die zweite Oberseite 6 die patientenabgewandte Oberseite sein, wobei die zweite Oberseite 6 des Basistuchelements das hydrophile Vliesmaterial aufweist. Das Basistuchelement 2 weist eine Schwächungslinie 14 auf, welche sich vom äußeren Rand 8 bis zum inneren Rand 10 erstreckt. Die Schwächungslinie 14 ist dabei eine alternierende Abfolge von Basistuchelement trennenden, insbesondere geradlinigen Schnitten einer Länge von 3-5 mm und von Basistuchelement verbindenden Bereichen, also Verbindungsstegen, insbesondere einer Länge von 0,4 - 1,0 mm. Die Schwächungslinie in dem ersten Bereich unterscheidet sich von der Schwächungslinie im zweiten Bereich. Entlang der Schwächungslinie 14 und die Schwächungslinie 14 überfangend ist ein erster Abdeckmaterialabschnitt 16 auf der ersten Oberseite 4 des Basistuchelements 2 zur Bildung eines ersten Lagenverbundes 30 flächenhaft festgelegt, wie dies Figur 1 zusammen mit der Figur 4 zeigt. Figur 4 zeigt schematisch eine Schnittansicht I -I aus der Figur 1. Zur Bildung eines ersten Lagenverbundes 30 ist ein Abdeckmaterialabschnitt 16 mittels eines Fügemittels 34 auf der ersten Oberseite 4 festgelegt. Der erste Abdeckmaterialabschnitt 16 ist vorzugsweise aus einem polymeren Flachmaterial mit einer Haftkleberbeschichtung gebildet, vorzugsweise wird als erster Abdeckmaterialabschnitt eine Polyurethanfolie mit einem einseitig aufgebrachten Acrylatkleber verwendet. Der erste Abdeckmaterialabschnitt 16 weist keine Schwächungslinie auf. Die innerhalb des ersten Lagenverbundes 30 dem ersten Abdeckmaterialabschnitt 16 zugewandte erste Oberseite 4 des Basistuchelements weist somit das Folienmaterial des Vlies-Folien-Laminats auf. In einer bevorzugten Ausführungsform ist eine Einreißkerbe 20, 22 ist in den ersten Lagenverbund eingebracht, und zwar ist vorzugsweise eine Einreißkerbe sowohl an dem äußeren Rand 8 als auch an dem inneren Rand 10 in der Flucht der Schwächungslinie 14 angeordnet. Der erste Lagenverbund 30 kann aber nach einer alternativen Ausführungsform nur an einem Rand, insbesondere nur am äußeren Rand 8 die Einreißkerbe 20 aufweisen (nicht in Figur 1 abgebildet). Die Einreißkerbe ist ein ca. 1 cm langer Einschnitt in den ersten Lagenverbund 30. Alternativ kann keine Einreißkerbe vorgesehen sein (nicht in Figur 1 abgebildet).

Figur 2 zeigt schematisch eine weitere Ausführungsform der erfindungsgemäßen OP-Abdeckung. Die Schwächungslinie 14 ist in dem ersten Bereich 24 des Basistuchelements 2 angeordnet. Desweiteren weist das Basistuchelement 2 eine Schnittlinie 28 auf. Die Schnittlinie 28 ist dabei im zweiten Bereich 26 angeordnet, und zwar in der Flucht der Schwächungslinie 14, und die Schnittlinie 28 erstreckt sich bis zu dem inneren Rand 10, der den Lochausschnitt begrenzt. Wie bereits zu Figur 1 erläutert, so ist auch bei dieser Ausführungsform die Schwächungslinie 14 mit einem ersten Abdeckmaterialabschnitt 16 überfangen, wobei der erste Abdeckmaterialabschnitt 16 auf der ersten Oberseite 4 des Basistuchelements zur Bildung eines ersten Lagenverbundes 30 flächenhaft festgelegt ist. Ein zweiter Abdeckmaterialabschnitt 18 überfängt die im zweiten Bereich 26 vorhandene Schnittlinie 28 und ist ebenso auf der ersten Oberseite 4 des Basistuchelements zur Bildung eines zweiten Lagenverbundes 32 mittels eines Fügemittels 34, insbesondere mittels eines Klebemittels flächenhaft festgelegt, wie in der Figur 5 als schematische Schnittansicht II-II der Figur 2 dargestellt ist. Auch der zweite Abdeckmaterialabschnitt 18 weist keine Schwächungslinie auf. Der zweite Abdeckmaterialabschnitt 18 besteht aus dem gleichen polymeren Material wie der voranstehend beschriebene erste Abdeckmaterialabschnitt 16, und weist das gleiche adhäsive Fügemittel in Form der am Abdeckmaterialabschnitt aufgebrachten Haftkleberbeschichtung auf. Der erste und zweite Abdeckmaterialabschnitt 16, 18 sind vorzugsweise aus einem einzigen Materialabschnitt gebildet, vorzugsweise aus einem Polyurethanfilm mit einer Acrylatkleberbeschichtung.

Figur 3 zeigt eine weitere Variante der erfindungsgemäßen OP-Abdeckung. Im Unterschied zu Figur 2 ist hierbei die Schwächungslinie 14 im zweiten Bereich 26 ausgehend vom inneren Rand 10 des Basistuchelements 2 angeordnet. Eine Schnittlinie 28 ist in der Flucht der Schwächungslinie 14 im ersten Bereich 24 des Basistuchelements angeordnet und erstreckt sich dabei bis zum äußeren Rand 8 des Basistuchelements. Die Schwächungslinie 14 und die Schnittlinie 28 sind auf der ersten Oberseite 4 von einem ersten Abdeckmaterialabschnitt 16 und von einem zweiten Abdeckmaterialabschnitt 18 überfangen, wobei der erste und zweite Abdeckmaterialabschnitt einen entsprechenden ersten und zweiten Lagenverbund mit dem Basistuchelement bilden. Der erste und zweite Abdeckmaterialabschnitt sind vorzugsweise aus einem einzigen Materialabschnitt gebildet. In einer bevorzugten Ausführungsform ist eine Einreißkerbe 22 in den ersten Lagenverbund am inneren Rand 10 eingebracht.

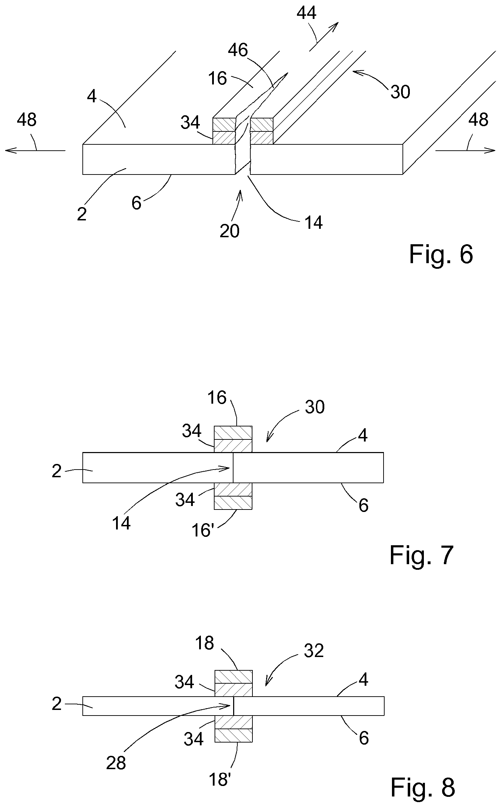

Die Figur 7 zeigt schematisch eine Schnittansicht eines Basistuchelements 2 einer besonderen Ausführungsform einer wegwerfbaren OP-Abdeckung, bei welcher ein weiterer erster Abdeckmaterialabschnitt 16' auf der anderen, nämlich der zweiten Oberseite 6, und die Schwächungslinie 14 dabei überfangend mittels eines Fügemittels 34 festgelegt ist. Bei dieser Ausführung umfasst der erste Lagenverbund 30 den ersten Abdeckmaterialabschnitt 16, das Fügemittel 34, das Basistuchelement 2, das Fügemittel 34 und den weiteren ersten Abdeckmaterialabschnitt 16'. Es kann auch vorgesehen sein, die bereits auf der ersten Oberseite 4 mittels eines zweiten Abdeckmaterialabschnittes 18 überfangende Schnittlinie 28 mit einem weiteren zweiten Abdeckmaterialabschnitt 18' auf der zweiten Oberseite 6 zu überfangen, wie Figur 8 schematisch zeigt. Dann umfasst der zweite Lagenverbund 32 den zweiten Abdeckmaterialabschnitt 18, das Fügemittel 34, das Basistuchelement 2, das Fügemittel 34 und den weiteren zweiten Abdeckmaterialabschnitt 18'. Die der ersten und zweiten Oberseite in einem jeweiligen ersten und/oder zweiten Lagenverbund zugeordneten ersten und/oder zweiten Abdeckmaterialabschnitte können identisch, deckungsgleich, aber alternativ auch unterschiedlich ausgestaltet sein.

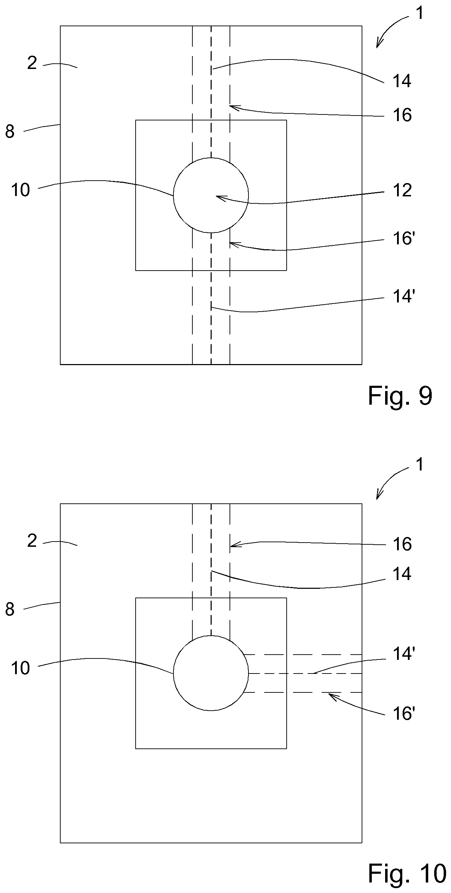

Eine erfindungsgemäße OP-Abdeckung 1 kann für eine weiter erleichterte Öffnungsmöglichkeit mit mehr als nur einer Schwächungslinie und einer gegebenenfalls in der Flucht dazu weitergeführten Schnittlinie ausgestattet sein. Die Figuren 9 und 10 zeigen schematisch eine weitere Ausführungsform der erfindungsgemäßen OP-Abdeckung mit zwei Schwächungslinien 14, 14', welche einem einzigen Lochausschnitt 12 zugeordnet sind. Die Schwächungslinien 14, 14' erstrecken sich jeweils vom äußeren Rand 8 bis zum inneren Rand 10. Die Schwächungslinien 14, 14' sind von jeweils einem ersten Abdeckmaterialabschnitt 16, 16' überfangen. Die ersten Abdeckmaterialabschnitte 16, 16' sind auf einer der Oberseiten des Basistuchelements 2 unter Ausbildung jeweils eines ersten Lagenverbundes flächenhaft festgelegt. Die beiden Schwächungslinien 14, 14' sind bevorzugt, wie in Figur 9 dargestellt ist, in einer Linie, also ein einem Winkel von 180° sich zueinander erstreckend, angeordnet. In einer weiteren bevorzugten alternativen Ausführung, wie in Figur 10 schematisch dargestellt ist, erstrecken sich die beiden Schwächungslinien 14, 14' zueinander in einem Winkel von 90°.

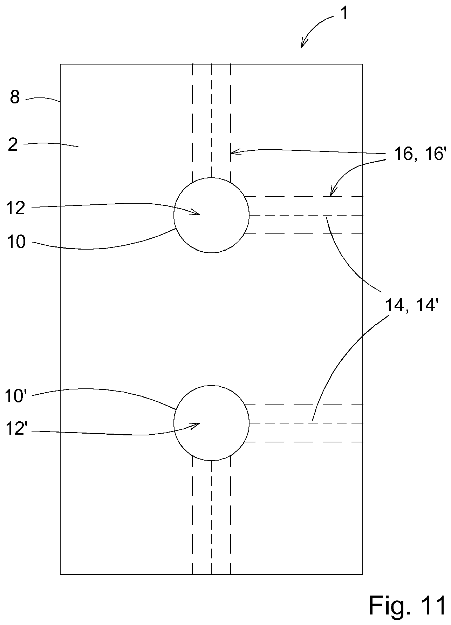

Figur 11 zeigt schematisch eine weitere Ausführungsform einer wegwerfbaren OP-Abdeckung 1. Dabei weist das Basistuchelement 2 zwei Lochausschnitte 12, 12' auf, welche innerhalb des äußeren Randes 8 des Basistuchelements angeordnet sind und jeweils von einem inneren Rand 10, 10' begrenzt sind. Beiden Lochausschnitten ist eine Öffnungsmöglichkeit in Form von jeweils zwei, vorzugsweise in einem Winkel von 90° angeordneten Schwächungslinien 14,14' zugeordnet. Vorzugsweise sind alle Schwächungslinien von einem Abdeckmaterialabschnitt 16, 16' überfangen. Die Abdeckmaterialabschnitte sind auf der ersten und/oder zweiten Oberseite des Basistuchelements unter Bildung von jeweils einem ersten Lagenverbund flächenhaft festgelegt.

Die OP-Abdeckungen 1, wie sie in den Figuren 1 bis 3 oder auch Figuren 9 bis 11 schematisch dargestellt sind, können ohne Einsatz von mechanischen Hilfsmitteln geöffnet werden, wie die Figur 6 schematisch zeigt. Ausgehend von einem Rand ist der erste Lagenverbund 30 entlang der Schwächungslinie 14 aufreißbar (mit einem Pfeil 44 angedeutet). Insbesondere vorteilhafthaft ist der erste Lagenverbund ausgehend von einer dem ersten Lagenverbund 30 zugeordneten Einreißkerbe 20 einreißbar. Der auf der ersten Oberseite 4 des Basistuchelements 2 mittels eines Fügemittels 34 flächenhaft festgelegte erste Abdeckmaterialabschnitt 16 löst sich beim Aufreißen des ersten Lagenverbundes 30 entlang der Schwächungslinie 14 dabei nicht vom Basistuchelement 2 ab. Damit ist eine Öffenbarkeit der OP-Abdeckung in einem Schritt gegeben, bei dem zudem auch keine Komponenten, wie der Abdeckmaterialabschnitt, vereinzelt werden, welche ansonsten entsorgt werden müssten. Die Öffenbarkeit dieser OP-Abdeckung 1 ohne Einsatz von mechanischen Hilfsmitteln ist durch eine Krafteinwirkung auf die OP-Abdeckung gegeben, und zwar ausgehend vom äußeren Rand 8, vorzugsweise ausgehend von der am Rand angeordneten Einreißkerbe 20 in Richtung und entlang der Schwächungslinie, so wie es in Figur 6 mit dem Pfeil 44 angedeutet ist. Bei Krafteinwirkung entsteht eine Einreißlinie 46 durch den Lagenverbund 30. Durch eine fortführende Krafteinwirkung und die damit entstehende fortführende Einreißlinie 46 bis hin zum jeweiligen anderen Rand (hier in Figur 6 nicht eingezeichnet) werden das Basistuchelement und damit die jeweilig darauf angeordneten Lagenverbunde geöffnet und die OP-Abdeckung kann entfernt werden. Zum Aufreißen des jeweiligen Lagenverbundes braucht es eine Krafteinwirkung. Für das gewollte bewusste Öffnen muss ausgehend vom Rand, insbesondere von der Einreißkerbe eine gewisse Weiterreißkraft eingesetzt werden. Die Weiterreißkraft wirkt dabei wie in dem nachfolgenden Versuchsaufbau zur Messung der Weiterreißkraft dargestellt ist. Anderseits ist ein Widerstand gewünscht, um hier eine Materialfestigkeit der OP-Abdeckung bereitstellen zu können. Die Materialfestigkeit zeigt sich u.a. in der Zugfestigkeit bei Krafteinwirkungen, die in der Ebene der Schwächungslinie bzw. des Lagenverbundes und dabei senkrecht auf die Schwächungslinie bzw. den Lagenverbund ausgeübt werden, wie mit Pfeil 48 angedeutet ist.

Nachfolgend sind die verwendeten Messmethoden im Detail beschrieben.

Methode mittlere Weiterreißkraft:

Die Methode der Messung der Weiterreißkraft ist an die DIN EN ISO 13937 - 2 von Juni 2000 angelehnt.

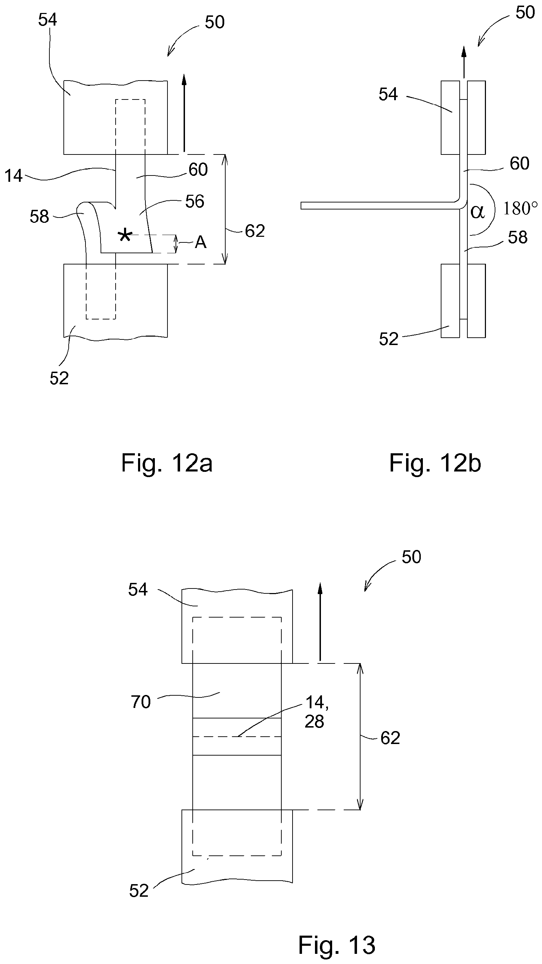

Es wird hierfür eine Zugprüfmaschine mit konstanter Prüfgeschwindigkeit eingesetzt. Es können Zugprüfmaschinen eingesetzt werden, bei der eine Einspannvorrichtung feststeht, während die andere sich während der Prüfung mit konstanter Geschwindigkeit bewegt und deren Lastrahmen keinerlei Durchbiegung aufweist. Eine solche Zugprüfmaschine ist das Prüfgerät Zwick Roell Typ Z005 von der Firma Zwick GmbH&Co.KG, Ulm, Deutschland. Die Zugprüfmaschine, nachfolgend als Prüfgerät bezeichnet, ist nur ausschnittsweise, also mit den für die unmittelbare Versuchsdurchführung relevanten Bereichen in den Figuren 12a und 12b schematisch dargestellt. Das Prüfgerät 50 umfasst u. a. eine untere, feststehende Einspannvorrichtung 52 und eine obere, bewegliche Einspannvorrichtung 54, deren Geschwindigkeit, die sogenannte Prüfgeschwindigkeit, mittels eines Steuerungsprogramms eingestellt wird. Die obere Einspannrichtung ist in vertikaler Richtung beweglich, wobei bei der Versuchsdurchführung der Weiterreißkraft diese obere Einspannrichtung vertikal nach oben gefahren wird, wie mit dem Pfeil angedeutet ist. In dieser Prüfung der Weiterreißkraft wird der benötigte Winkel von 180° ausschließlich durch die beiden Einspannvorrichtungen, die untere feststehende und die obere bewegliche Klemme, bereitgestellt.

Der Versuch ist schematisch in Figur 12a und 12b dargestellt. Zur Messung der Weiterreißkraft in einem Lagenverbund entlang einer Schwächungslinie 14 wird ein Prüfling 56 mit den Abmessungen von 50 mm Probenbreite und 100 mm Probenlänge aus der OP-Abdeckung mit einer durch einen Abdeckmaterialabschnitt überfangenen Schwächungslinie ausgestanzt, derart, dass die Schwächungslinie 14 dabei mittig in Längsrichtung des Prüflings angeordnet ist. Analog werden die Prüflinge zur Messung der Weiterreißkraft in einem zweiten Lagenverbund entlang einer Schnittlinie vorbereitet.

Für die Durchführung der Prüfung werden die Prüflinge vorher 24 Stunden im Normklima (23 °C, 50 % relative Luftfeuchtigkeit) konditioniert. Der Prüfling 56 wird entlang der Schwächungslinie bzw. der Schnittlinie beginnend an einem Rand des Prüflings über eine Länge von 50 mm eingeschnitten, so dass zwei Schenkel 58, 60 entstehen. Ein Schenkel 58 wird in eine untere Einspannvorrichtung 52, der andere Schenkel 50 in die obere, bewegliche Einspannvorrichtung 54 eingespannt. Die Einspannlänge 62 beträgt 50 mm und wird verstanden als der Abstand zwischen den beiden effektiven Klemmpunkten der Prüfeinrichtung. Eine Vorspannung bei Beginn der Prüfung muss vermieden werden. Die Schenkel müssen dabei schlupffrei festgehalten werden, ohne diese abzuscheren oder in irgendeiner anderen Form zu beschädigen. Die Klemmflächen dürfen dabei nicht schmaler als der Prüfling sein. Mit einer Prüfgeschwindigkeit von 500 mm/min werden die obere bewegliche Einspannvorrichtung 54 (in Fig. 12a und Fig. 12b mit einem Pfeil angedeutet) und damit der darin fixierte Schenkel 60 gezogen. Durch die Bewegung der oberen Einspannvorrichtung 54 stellt sich der Abzugswinkel der beiden Schenkel zueinander von 180° ein (Fig.12b), und der Prüfling wird dabei getrennt. Die Trennung wird bis zu einem verbleibenden Abstand A von 25 mm zum gegenüberliegenden Seitenrand des Prüflings durchgeführt. Für die Auswertung wird das arithmetische Mittel der über den Messweg von 25 mm ermittelten Kraftspitzen als die mittlere Weiterreißkraft bestimmt. Es werden Einzelmessungen an fünf Prüflingen durchgeführt und daraus wird wiederum der Mittelwert bestimmt.

Für das Aufreißen des ersten Lagenverbundes 30 entlang der Schwächungslinie 14 in der nach Figur 1 beschriebenen OP-Abdeckung wurde eine mittlere Weiterreißkraft von 9-11 N ermittelt. In einer analogen Prüfung wird die mittlere Weiterreißkraft für das Aufreißen des zweiten Lagenverbundes 32 entlang der Schnittlinie 28 analysiert. Hierzu wurde eine mittlere Weiterreißkraft von 3-6 N ermittelt.

Methode Zugfestigkeit des Lagenverbundes

Diese Prüfung dient der Ermittlung der Beziehung zwischen Zugkraft und Längenänderung von streifenförmigen Prüflingen, die aus einem jeweiligen Lagenverbund, also dem Bereich mit einer mit einem Abdeckmaterialabschnitt überfangenden Schwächungslinie bzw. überfangenden Schnittlinie, entnommen sind. Die Prüfung ist an EN 29073-3: 1992 angelehnt.

Es werden dazu Prüflinge mit einer Abmessung von 260 mm x 50 mm ausgestanzt, derart dass die Schwächungslinie bzw. Schnittlinie parallel zu der kürzeren Kante und mittig der längeren Kante des Prüflings angeordnet ist. Die Prüflinge werden vor der Prüfung mindestens 24 Stunden im Normklima (23 °C, 50 % relative Luftfeuchtigkeit) konditioniert. Für die Prüfung der Zugfestigkeit wird eine Zugprüfmaschine, wie vorstehend bei der Messung der Weiterreißkraft beschrieben, eingesetzt. Die Zugprüfmaschine, nachfolgend als Prüfgerät 50 bezeichnet, weist eine untere Einspannvorrichtung 52 und eine obere und vertikal nach oben bewegbare (wie in Figur 13 mit Pfeil angedeutet) Einspannvorrichtung 54 auf.

Mit einer Einspannlänge 62 von 50 mm, also dem Abstand zwischen der unteren festgestellten Einspannvorrichtung 52 und der oberen, beweglichen Einspannvorrichtung 54, wird der Prüfling 70 mit der jeweiligen kurzen Kante in die untere bzw. die obere Einspannvorrichtung befestigt, wobei die Schwächungslinie 14 bzw. die Schnittlinie 28 mittig der Einspannlänge 62 platziert wird. Es wird eine Vorkraft von 0,2N angelegt. Bei einer konstanten Geschwindigkeit der oberen Einspannvorrichtung 54 von 100 mm/min wird eine Kraft-Dehnungskurve aufgezeichnet. Hieraus wird das erste Kraftmaximum ermittelt. Es werden Einzelmessungen an drei Prüflingen durchgeführt und daraus der Mittelwert ermittelt. Die Kraftwerte werden in der Einheit N/50mm angegeben.

Der erste Lagenverbund in der nach Figur 1 beschriebenen Ausführung der OP-Abdeckung weist eine Zugfestigkeit von 40 - 50N/50mm auf.

Methode Reißkraft und Reißdehnung des polymeren Abdeckmaterialabschnittes:

Die Methode dient der Bestimmung der Festigkeit und Dehnbarkeit von Folienmaterialien, wie die eines polymeren Abdeckmaterialabschnittes.

Dafür werden Prüflinge mit sauberen Schnittkanten von 25 mm Breite und 250 mm Länge aus der Folienbahn entnommen. Bei Folienwerkstoffen, deren Eigenschaften sich mit der Richtung der Ebene der Folie ändern, ist es notwendig, Prüflinge sowohl in Maschinenrichtung (MD) als auch gegen die Maschinenrichtung (CD) der Folie herzustellen. Die Prüflinge werden spannungslos über mindestens 24 h bei 23°C und 50% relativer Luftfeuchtigkeit klimatisiert. Anschließend wird der Prüfling in eine vertikale Zugprüfmaschine, wie vorstehend bei der Messung der Weiterreißkraft beschrieben, eingespannt, wobei der Abstand der Einspannvorrichtungen, also die Einspannlänge L0 50 mm für die Messung der Prüflinge in CD-Richtung und L0 100 mm für die Messung der Prüflinge in MD-Richtung beträgt. Die bewegliche Einspannvorrichtung wird mit einer Geschwindigkeit von 500 mm/min bewegt bis der Prüfling die maximale Kraft zum ersten Reißen erreicht hat. Anschließend wird die gedehnte Länge L1 des Abschnittes in mm gemessen.

Bei dieser Messung kann der Messpunkt des ersten Reißens, also die erste vermessene Kraftspitze, die Reißkraft ermittelt werden. Diese Kraft, ausgedrückt in N wird für die beide Hauptachsen des Folienmaterials, also MD und CD, ermittelt.

Die Dehnung in % berechnet sich dann nach folgender Formel:

$$Dehnung[\%] = \frac{L1[mm] - L0[mm]}{L0[mm]} x100\%$$

Die Reißdehnung wird als die am Messpunkt der Reißkraft maximal erreichte Dehnung (ausgedrückt in %) verstanden. Diese Reißdehnung wird ebenso für MD- und CD-Richtung ermittelt. Es werden Einzelmessungen an je fünf Prüflingen für MD- und CD-Richtung durchgeführt.

Der in der Ausführungsform der OP-Abdeckung nach Figur 1 eingesetzte Abdeckmaterialabschnitt aus polymerem Material, nämlich ein Polyurethanfilm weist eine Reißkraft von 20-30 mm/25mm in MD-Richtung und von 20-30 mm/25mm in CD -Richtung und eine Reißdehnung von 450-650% in MD-Richtung und von 650-850% in CD-Richtung auf.

Methode Delaminierungswiderstand

Diese Methode dient der Beurteilung, wie hoch die Trennkraft von miteinander verbundenen Materiallagen ist, also im vorliegenden Fall der Trennung von Abdeckmaterialabschnitt vom Basistuchelement. Die Prüfung ist an die DIN 53 357, Verfahren A angelehnt.

Es werden dazu Prüflinge mit der Abmessung von 50 mm Breite und 260 mm Länge ausgestanzt. Die beiden Schichten des Prüflings, d.h. das Basistuchelement und der Abdeckmaterialabschnitt werden ausgehend von einer beliebigen Schmalseite des Prüflings auf eine Länge von mindestens 40 mm nach Möglichkeit manuell voneinander getrennt. Dabei sollen die Schichten möglichst nicht verletzt werden. Können die Schichten manuell nicht getrennt werden, kann dies durch das Benetzen mit einem mit Lösungsmittel getrennten Lappen erleichtert werden. Die so vorbereiteten Prüflingen werden mindestens 16 Stunden im Normklima gelagert (23 °C, 50 % relative Luftfeuchtigkeit). Die aufgetrennten Schichten des Prüflings werden in jeweils die obere bzw. die untere Einspannvorrichtung einer Zugprüfmaschine, ein Prüfgerät 50, wie bei der Methode der Weiterreißkraft oder der Zugfestigkeit beschrieben ist, eingespannt. Die Einspannlänge, also der Abstand zwischen der unteren und der oberen Einspannvorrichtung beträgt 50 mm. Mit einer Prüfgeschwindigkeit von 300 mm/min wird die obere Einspannvorrichtung vertikal nach oben weggezogen, was zu einer Trennung der Schichten führt. Wichtig ist, dass das noch nicht getrennte Ende des Prüflings rechtwinklig zur Zugrichtung gehalten wird. Bei der Auswertung sind ein Vorweg von 10 mm, der eigentliche Messweg von 100 mm und ein Nachlaufweg von 10 mm zu beachten. Der Delaminierungswiderstand ist hierbei die über die Länge des Messwegs ermittelte mittlere Kraft. Für die Auswertung wird das Ergebnis in der Einheit N bezogen auf die Probenbreite von 50 mm angegeben. Aus fünf Einzelmessungen wird der Mittelwert errechnet.

Die in Figur 1 beschriebene OP-Abdeckung weist einen Delaminierungswiderstand zwischen Abdeckmaterialabschnitt und Basistuchelement mit einer mittleren Kraft von 2-3N/50 mm auf.

Methode Durchdringungswiderstand

Mit dieser Methode wird in einem hydrostatischen Druckversuch der Widerstand gegen das Durchdringen von Wasser eines Lagenverbundes, also eines mittels eines Abdeckungsmaterialabschnittes überfangenden Bereiches der Schwächungslinie bzw. der Schnittlinie festgestellt. Die Methode wird in Anlehnung an die Norm EN 20811 durchgeführt. Als Prüfgerät wird ein Wasserdichtigkeitsprüfung Gerät nach EN 20811 mit einer Prüffläche von 100 cm², z.B. FX3000 der Firma TEXTEST Instruments, eingesetzt.

Zur Probenvorbereitung werden mindestens fünf Prüflinge von verschiedenen Stellen eines Lagenverbundes eingesetzt. Die Prüflinge werden vor Durchführung der Prüfung mindestens 24 Stunden im Normklima (23° C/ 50 % relative Luftfeuchtigkeit) konditioniert. Bei der Durchführung der Prüfung wird für jede zu prüfende Messprobe ein frisch destilliertes Wasser verwendet. Der jeweilige Prüfling wird so in den Prüfkopf der Prüfvorrichtung eingespannt, dass die Schwächungslinie bzw. die Schnittlinie mittig innerhalb der Prüffläche des Prüfgerätes angeordnet ist, und die Oberfläche des Prüflings mit dem Wasser in Berührung kommt. Das Einspannen der Prüflinge erfolgt jedoch so, dass vor Prüfungsbeginn noch kein Wasser durch den Prüfling treten kann. Der Prüfling wird hierbei so eingespannt, dass die Oberseite mit dem darauf angebrachten Abdeckmaterialabschnitt der Wasseroberfläche zugewandt ist. Der Prüfling wird dann einem stetig steigenden Wasserdruck ausgesetzt, hierbei mit einer Steigungsgeschwindigkeit des Wasserdruckes von 10 mbar/min. Der Wasserdruck wird dabei von unten auf den Prüfling ausgeübt. Die Temperatur des für die Messung eingesetzten Wassers beträgt 20 °CEs wird der Druck in mbar registriert, bei dem das Wasser zuerst an der dritten Stelle des Prüflings austritt. Kleine Tröpfchen, die sich nach ihrer Bildung nicht mehr vergrößern, werden vernachlässigt. Es werden keine Tropfen berücksichtigt, die anschließend durch dieselbe Stelle des Prüflings durchdringen. In Abwandlung der EN 20811 wird unter Durchdringungswiderstand dabei auch der Druck in mbar bis zu dem Wert registriert, solange das Wasser gehalten und es dann anschließend zu einem Bersten des Prüflings kommt. Bei der Prüfung des Durchdringungswiderstandes werden mindestens 3 Prüflinge vermessen.

Bei der OP-Abdeckung wurde unter den vorgenannten Prüfbedingungen stets ein Durchdringungswiderstand von größer 30 mbar festgestellt.

Methode Haftkraft 180° Peel gegenüber einer Polyethylenoberfläche

Bei dieser Prüfung wird die Kraft gemessen, welche erforderlich ist, um einen Abdeckmaterialabschnitt mit einer Kleberbeschichtung von einer planen definierten Oberfläche in einem Winkel von 180° mit konstanter Geschwindigkeit abzuziehen.

Dazu werden Prüflinge in der Größe von 250 mm Länge und 25 mm Breite aus einem entsprechenden Flachbahnmaterial mit Kleberbeschichtung ausgestanzt.

Die Prüflinge wurden vor der Versuchsdurchführung 24 h im Normklima (23°C, 50 % relative Luftfeuchtigkeit) gelagert.

Als Polyethylenoberfläche wird eine PE-Prüfplatte der Firma Rocholl, Fabrikat Simona HWU, schwarz, Abmessung 150 x 50 x 2 mm eingesetzt. Die PE-Platte weist eine glänzende und eine matte Oberseite auf. Der Prüfling wird mit der den Klebstoff aufweisenden Oberseite in seiner Längsrichtung mittig längs auf die PE-Prüfplatte und zwar auf dessen glänzende Oberseite, aufgelegt und insgesamt 4x mit einem Druck von 20N/cm mittels eines Handrollers überrollt. Nach einer Wartezeit von 10 Minuten erfolgt die Messung.

Dafür wird die Prüfplatte in die untere, feststehende Einspanneinrichtung (Klemme) einer Zugprüfmaschine und der der Prüfling wird mit seinem freien Ende in die obere Klemme des Zugprüfgerätes so eingespannt, dass ein 180°-Winkel vorliegt. Die Einspannlänge, also der Abstand zwischen Prüfplatte und oberer Klemme beträgt 20 mm. Dann wird der Prüfling mit einer Geschwindigkeit von 300 mm/min von der Oberseite des Prüfsubstrats abgezogen. Der Messweg beträgt 50 mm, der Vor- und Nachmessweg beträgt jeweils 25 mm.

Die während des Messweges auftretenden Kräfte werden durch eine an der Zugprüfmaschine angeschlossene Auswerteeinheit aufgezeichnet. Die Kraft F (max) stellt dabei die maximale Kraftspitze während des Messweges dar; Kraft F (mittel) den Mittelwert des Kraftverlaufs über den Messweg. Für die Auswertung wird das Ergebnis in der Einheit N bezogen auf die Probenbreite von 25 mm angegeben. Es werden 3 einzelne Prüflinge vermessen.

Die Haftkraft F (mittel) des in der nach Figur 1 beschriebenen OP-Abdeckung eingesetzten Abdeckmaterialabschnitts, nämlich eine Polyurethanfolie mit einer Acrylatbeschichtung, beträgt mindestens 4N/25mm.

**Patentansprüche**

1. Wegwerfbare OP-Abdeckung (1) umfassend ein flüssigkeitsundurchlässiges Basistuchelement (2) mit einer ersten und einer zweiten Oberseite (4, 6), mit einem äußeren Rand (8) und einem inneren Rand (10), wobei der innere Rand (10) mindestens einen innerhalb des äußeren Randes (8) des Basistuchelements (2) angeordneten Lochausschnitt (12) begrenzt, wobei das Basistuchelement (2) mindestens eine Schwächungslinie (14) aufweist, die sich ausgehend von dem äußeren Rand (8) des Basistuchelements (2) in Richtung des inneren Randes (10) oder die sich ausgehend von dem inneren Rand (10) in Richtung des äußeren Randes (8) erstreckt, wobei ein erster Abdeckmaterialabschnitt (16) entlang der Schwächungslinie (14) und die Schwächungslinie (14) dabei überfangend auf der ersten und/oder zweiten Oberseite des Basistuchelements zur Bildung eines ersten Lagenverbundes (30) flächenhaft festgelegt ist, wobei der erste Abdeckmaterialabschnitt (16) keine Schwächungslinie aufweist, und der erste Lagenverbund (30) ausgehend vom äußeren Rand (8) und/oder vom inneren Rand (10) entlang der Schwächungslinie (14) aufreißbar ist, ohne dass sich der erste Abdeckmaterialabschnitt (16) von dem Basistuchelement (2) löst, wobei das Basistuchelement (2) einen an den äußeren Rand (8) angrenzenden äußeren ersten Bereich (24) und einen an den ersten Bereich (24) angrenzenden und sich nach innen zu dem Lochausschnitt (12) erstreckenden inneren zweiten Bereich (26) aufweist, **dadurch gekennzeichnet, dass** die Schwächungslinie (14) sich über den ersten Bereich (24) hinweg bis in den zweiten Bereich (26) hinein erstreckt, wobei die Schwächungslinie (14) in dem ersten Bereich sich von der Schwächungslinie (14) in dem zweiten Bereich unterscheidet, wobei die Unterschiede in der Schwächungslinie durch die Ausgestaltung der Basistuchelement trennenden Bereiche und die das Basistuchelement verbindenden Bereiche gebildet sind; oder dass die Schwächungslinie (14) in dem ersten Bereich (24) angeordnet ist und dass das Basistuchelement (2) in dem zweiten Bereich (26) eine Schnittlinie (28) aufweist, die in der Flucht der Schwächungslinie (14) angeordnet ist, wobei die Schnittlinie (28) sich in Richtung zu dem inneren Rand (10), vorzugsweise bis zu dem inneren Rand (10) des Basistuchelements (2) erstreckt; oder dass die Schwächungslinie (14) in dem zweiten Bereich (26) angeordnet ist und dass das Basistuchelement in dem ersten Bereich (24) eine Schnittlinie (28) aufweist, die in der Flucht der Schwächungslinie (14) angeordnet ist, wobei die Schnittlinie (28) sich in Richtung zu dem äußeren Rand (8), vorzugsweise bis zu dem äußeren Rand (8) des Basistuchelements (2) erstreckt.

2. Wegwerfbare OP-Abdeckung nach Anspruch 1, **dadurch gekennzeichnet, dass** das Basistuchelement zwei, dem Lochausschnitt (12) zugeordnete Schwächungslinien (14, 14') aufweist, die insbesondere in einem Winkel von 90° sich zueinander erstreckend oder in einer Linie, also in einem Winkel von 180° sich zueinander erstreckend, angeordnet sind.

3. Wegwerfbare OP-Abdeckung nach Anspruch 1 oder 2, **dadurch gekennzeichnet, dass** der erste Lagenverbund (30) eine Einreißkerbe (20, 22) an dem äußeren Rand (8) und/oder an dem inneren Rand (10) aufweist, derart, dass die Einreißkerbe (20, 22) in der Flucht der Schwächungslinie (14) angeordnet ist, und der erste Lagenverbund (30) ausgehend von der Einreißkerbe (20, 22) entlang der Schwächungslinie (14) aufreißbar ist.

4. Wegwerfbare OP-Abdeckung (1) nach einem der Ansprüche 1 bis 3, **dadurch gekennzeichnet, dass** der erste Bereich (24) und der zweite Bereich (26) sich zumindest in einer Eigenschaft unterscheiden, mit den Eigenschaften entnommen aus der Gruppe Materialart, Lagenanzahl oder Materialeigenschaften, wie Dicke, Flächengewicht, Zugfestigkeit, Dehnbarkeit, Reißfestigkeit, Drapierbarkeit, Atmungsaktivität, Absorptionskapazität, Farbe oder Transparenz.

5. Wegwerfbare OP-Abdeckung (1) nach einem der Ansprüche 1 bis 4, **dadurch gekennzeichnet, dass** ein zweiter Abdeckmaterialabschnitt (18) entlang der Schnittlinie (28) und die Schnittlinie (28) dabei überfangend auf der ersten und/oder zweite Oberseite des Basistuchelements zur Bildung eines zweiten Lagenverbundes (32) flächenhaft festgelegt ist und der zweite Lagenverbund (32) entlang der Schnittlinie (28) aufreißbar ist, ohne dass sich der zweite Abdeckmaterialabschnitt (18) von dem Basistuchelement löst.

6. Wegwerfbare OP-Abdeckung (1) nach Anspruch 5, **dadurch gekennzeichnet, dass** der zweite Abdeckmaterialabschnitt (18) keine Schwächungslinie aufweist.

7. Wegwerfbare OP-Abdeckung (1) nach Anspruch 5 oder 6, **dadurch gekennzeichnet, dass** der erste und zweite Abdeckmaterialabschnitt (16,18) aus einem einzigen Materialabschnitt gebildet sind.

8. Wegwerfbare OP-Abdeckung (1) nach einem der Ansprüche 1 bis 7, **dadurch gekennzeichnet, dass** der erste und/oder der zweite Abdeckmaterialabschnitt (16, 18) mittels Fügemittel (34) entnommen aus der Gruppe Klebemittel, wie insbesondere doppelseitiges Klebeband oder Haftkleberbeschichtung, Schweißstellen, wie insbesondere Thermolaminierung, oder Thermobonding auf dem Basistuchelement festgelegt ist.

9. Wegwerfbare OP-Abdeckung (1) nach einem der Ansprüche 1 bis 8, **dadurch gekennzeichnet, dass** der erste und/oder der zweite Abdeckmaterialabschnitt (16, 18) aus einem polymeren Material besteht, insbesondere aus polymerem Material entnommen aus der Gruppe Polyethylen, Polypropylen, Polyurethan oder Mischungen davon.

10. Wegwerfbare OP-Abdeckung (1) nach Anspruch 9, **dadurch gekennzeichnet, dass** der erste und zweite Abdeckmaterialabschnitt (16, 18) aus dem gleichen polymeren Material bestehen.

11. Wegwerfbare OP-Abdeckung (1) nach einem der Ansprüche 1 bis 10, **dadurch gekennzeichnet, dass** zumindest die innerhalb des ersten und/oder des zweiten Lagenverbundes (30, 32) dem Abdeckmaterialabschnitt (16, 18) zugewandte Oberseite (4, 6) des Basistuchelements, insbesondere die erste Oberseite (4) des Basistuchelements ein Folienmaterial aufweist, insbesondere dass die gesamte erste Oberseite (4) aus einem Folienmaterial gebildet ist.

12. Wegwerfbare OP-Abdeckung (1) nach einem der Ansprüche 1 bis 11, **dadurch gekennzeichnet, dass** die erste Oberseite (4) aus einem anderen Material oder einem anderen Materialverbund gebildet ist als die zweite Oberseite (6), und dass eine der Oberseiten (4, 6), insbesondere die zweite Oberseite (6) des Basistuchelements ein absorbierendes Material, insbesondere ein Vliesmaterial, insbesondere ein hydrophiles Vliesmaterial aufweist oder daraus gebildet ist.

13. Wegwerfbare OP-Abdeckung (1) nach einem der Ansprüche 1 bis 12, **dadurch gekennzeichnet, dass** der erste Bereich (24) ein Vlies-Folien-Laminat aufweist, insbesondere daraus gebildet ist und der zweite Bereich (26) eine insbesondere transparente Folie aufweist, insbesondere daraus gebildet ist.

14. Wegwerfbare OP-Abdeckung (1) nach einem der Ansprüche 1 bis 13, **dadurch gekennzeichnet, dass** ein weiterer erster und/oder zweiter Abdeckmaterialabschnitt (16', 18') auf einer Oberseite des Basistuchelements entlang der Schwächungslinie (14) und/oder der Schnittlinie (28) und die Schwächungslinie und/oder die Schnittlinie dabei überfangend festgelegt ist, derart, dass die Schwächungslinie und/oder die Schnittlinie sowohl auf der ersten als auch auf der zweiten Oberseite von einem Abdeckmaterialabschnitt (16, 18, 16',18') überfangen ist.

15. Wegwerfbare OP-Abdeckung (1) nach einem der Ansprüche 1 bis 14, **dadurch gekennzeichnet, dass** der erste und/oder zweite Abdeckmaterialabschnitt (16, 18, 16', 18') aus einem polymeren Flachmaterial gebildet ist, wobei eine Oberseite des Flachmaterials eine Haftkleberbeschichtung aufweist.

**Claims**

1. Disposable surgical drape (1) comprising a liquid-impermeable base cloth element (2) with a first and a second top side (4, 6), with an outer periphery (8) and an inner periphery (10), wherein the inner periphery (10) delimits at least one cutout hole (12) which is arranged within the outer periphery (8) of the base cloth element (2), wherein the base cloth element (2) has at least one weakening line (14) which extends from the outer periphery (8) of the base cloth element (2) in the direction of the inner periphery (10) or which extends from the inner periphery (10) in the direction of the outer periphery (8), wherein a first covering material portion (16) is areally fixed, along the weakening line (14) and so as to thereby cover the weakening line (14), on the first and/or second top side of the base cloth element in order to form a first layer composite (30), wherein the first covering material portion (16) has no weakening line, and the first layer composite (30) can be torn along the weakening line (14) proceeding from the outer periphery (8) and/or from the inner periphery (10), without the first covering material portion (16) becoming detached from the base cloth element (2), wherein the base cloth element (2) has an outer first region (24) adjoining the outer periphery (8) and an inner second region (26) adjoining the first region (24) and extending inwardly to the cutout hole (12), **characterized in that** the weakening line (14) extends beyond the first region (24) into the second region (26), wherein the weakening line (14) in the first region differs from the weakening line (14) in the second region, wherein the differences in the weakening line are formed by the design of the regions separating the base cloth element and the regions connecting the base cloth element, or **in that** the weakening line (14) is arranged in the first region (24) and **in that** the base cloth element (2) in the second region (26) has a cutting line (28) which is arranged in alignment with the weakening line (14), wherein the cutting line (28) extends in the direction of the inner periphery (10), preferably up to the inner periphery (10) of the base cloth element (2), or **in that** the weakening line (14) is arranged in the second region (26) and **in that** the base cloth element in the first region (24) has a cutting line (28) which is arranged in alignment with the weakening line (14), wherein the cutting line (28) extends in the direction of the outer periphery (8), preferably up to the outer periphery (8) of the base cloth element (2).

2. Disposable surgical drape according to Claim 1, **characterized in that** the base cloth element has two weakening lines (14, 14') which are assigned to the cutout hole (12) and which are arranged so as to extend in particular at an angle of 90° relative to one another or in a line, that is to say so as to extend at an angle of 180° relative to one another.

3. Disposable surgical drape according to Claim 1 or 2, **characterized in that** the first layer composite (30) has a tear notch (20, 22) on the outer periphery (8) and/or on the inner periphery (10), in such a way that the tear notch (20, 22) is arranged in alignment with the weakening line (14), and the first layer composite (30) can be torn along the weakening line (14) proceeding from the tear notch (20, 22).

4. Disposable surgical drape (1) according to one of Claims 1 to 3, **characterized in that** the first region (24) and the second region (26) differ at least in terms of one property, with the properties taken from the group of type of material, number of layers or material properties such as thickness, weight per unit area, tensile strength, extensibility, tear resistance, drapeability, breathability, absorption capacity, colour or transparency.

5. Disposable surgical drape (1) according to one of Claims 1 to 4, **characterized in that** a second covering material portion (18) is areally fixed, along the cutting line (28) and so as to thereby cover the cutting line (28), on the first and/or second top side of the base cloth element in order to form a second layer composite (32), and the second layer composite (32) can be torn along the cutting line (28), without the second covering material portion (18) becoming detached from the base cloth element.

6. Disposable surgical drape (1) according to Claim 5, **characterized in that** the second covering material portion (18) has no weakening line.

7. Disposable surgical drape (1) according to Claim 5 or 6, **characterized in that** the first and second covering material portions (16, 18) are formed from a single material portion.

8. Disposable surgical drape (1) according to one of Claims 1 to 7, **characterized in that** the first and/or the second covering material portion (16, 18) are/is fixed on the base cloth element by way of joining means (34) taken from the group of adhesive means, such as in particular double-sided adhesive tape or a pressure-sensitive adhesive coating, welded joints, such as in particular thermolamination, or thermobonding.

9. Disposable surgical drape (1) according to one of Claims 1 to 8, **characterized in that** the first and/or the second covering material portion (16, 18) are/is composed of a polymeric material, in particular of polymeric material taken from the group of polyethylene, polypropylene, polyurethane or mixtures thereof.

10. Disposable surgical drape (1) according to Claim 9, **characterized in that** the first and second covering material portions (16, 18) are composed of the same polymeric material.

11. Disposable surgical drape (1) according to one of Claims 1 to 10, **characterized in that** at least that top side (4, 6) of the base cloth element which faces towards the covering material portion (16, 18) within the first and/or the second layer composite (30, 32), in particular the first top side (4) of the base cloth element, comprises a film material, in particular **in that** the entire first top side (4) is formed from a film material.

12. Disposable surgical drape (1) according to one of Claims 1 to 11, **characterized in that** the first top side (4) is formed from a different material or a different material composite than the second top side (6), and **in that** one of the top sides (4, 6), in particular the second top side (6) of the base cloth element, comprises or is formed from an absorbent material, in particular a nonwoven material, in particular a hydrophilic nonwoven material.

13. Disposable surgical drape (1) according to one of Claims 1 to 12, **characterized in that** the first region (24) comprises a nonwoven-film laminate, in particular is formed therefrom, and the second region (26) comprises an in particular transparent film, in particular is formed therefrom.

14. Disposable surgical drape (1) according to one of Claims 1 to 13, **characterized in that** a further first and/or second covering material portion (16', 18') are/is fixed on a top side of the base cloth element, along the weakening line (14) and/or the cutting line (28) and so as to thereby cover the weakening line and/or the cutting line, in such a way that the weakening line and/or the cutting line are/is covered by a covering material portion (16, 18, 16', 18') both on the first and on the second top side.

15. Disposable surgical drape (1) according to one of Claims 1 to 14, **characterized in that** the first and/or second covering material portion (16, 18, 16', 18') are/is formed from a flat polymeric material, wherein a top side of the flat material has a pressure-sensitive adhesive coating.

**Revendications**

1. Champ opératoire à usage unique (1) comprenant un élément toile de base imperméable aux liquides (2) ayant un premier et un deuxième côté supérieur (4, 6), ayant un bord extérieur (8) et un bord intérieur (10), le bord intérieur (10) délimitant au moins une découpe de trou (12) agencée à l'intérieur du bord extérieur (8) de l'élément toile de base (2), l'élément toile de base (2) comprenant au moins une ligne d'affaiblissement (14), qui s'étend en direction du bord intérieur (10) en partant du bord extérieur (8) de l'élément toile de base (2) ou qui s'étend en direction du bord extérieur (8) en partant du bord intérieur (10), une première section de matériau de recouvrement (16) étant fixée à plat le long de la ligne d'affaiblissement (14) et doublant ainsi la ligne d'affaiblissement (14) sur le premier et/ou le deuxième côté supérieur de l'élément toile de base pour la formation d'un premier composite de couches (30), la première section de matériau de recouvrement (16) ne comprenant pas de ligne d'affaiblissement, et le premier composite de couches (30) pouvant être déchiré le long de la ligne d'affaiblissement (14) en partant du bord extérieur (8) et/ou du bord intérieur (10), sans que la première section de matériau de recouvrement (16) ne se détache de l'élément toile de base (2), l'élément toile de base (2) comprenant une première zone extérieure (24) adjacente au bord extérieur (8) et une deuxième zone intérieure (26) adjacente à la première zone (24) et s'étendant vers l'intérieur jusqu'à la découpe de trou (12), **caractérisé en ce que** la ligne d'affaiblissement (14) s'étend sur la première zone (24) jusque dans la deuxième zone (26), la ligne d'affaiblissement (14) dans la première zone différant de la ligne d'affaiblissement (14) dans la deuxième zone, les différences dans la ligne d'affaiblissement étant formées par la configuration des zones séparant l'élément toile de base et des zones reliant l'élément toile de base, ou **en ce que** la ligne d'affaiblissement (14) est agencée dans la première zone (24) et **en ce que** l'élément toile de base (2) comprend une ligne de coupe (28) dans la deuxième zone (26), qui est agencée en alignement avec la ligne d'affaiblissement (14), la ligne de coupe (28) s'étendant en direction du bord intérieur (10), de préférence jusqu'au bord intérieur (10) de l'élément toile de base (2), ou **en ce que** la ligne d'affaiblissement (14) est agencée dans la deuxième zone (26) et **en ce que** l'élément toile de base comprend dans la première zone (24) une ligne de coupe (28), qui est agencée en alignement avec la ligne d'affaiblissement (14), la ligne de coupe (28) s'étendant en direction du bord extérieur (8), de préférence jusqu'au bord extérieur (8) de l'élément toile de base (2).

2. Champ opératoire à usage unique selon la revendication 1, **caractérisé en ce que** l'élément toile de base comprend deux lignes d'affaiblissement (14, 14'), associées à la découpe de trou (12), qui sont notamment agencées en s'étendant à un angle de 90° l'une par rapport à l'autre ou en une ligne, c'est-à-dire en s'étendant à un angle de 180° l'une par rapport à l'autre.

3. Champ opératoire à usage unique selon la revendication 1 ou 2, **caractérisé en ce que** le premier composite de couches (30) comprend une entaille de déchirure (20, 22) sur le bord extérieur (8) et/ou sur le bord intérieur (10), de telle sorte que l'entaille de déchirure (20, 22) est agencée en alignement avec la ligne d'affaiblissement (14), et le premier composite de couches (30) peut être déchiré le long de la ligne d'affaiblissement (14) en partant de l'entaille de déchirure (20, 22).

4. Champ opératoire à usage unique (1) selon l'une quelconque des revendications 1 à 3, **caractérisé en ce que** la première zone (24) et la deuxième zone (26) diffèrent au moins au niveau d'une propriété, la propriété étant extraite du groupe constitué par le type de matériau, le nombre de couches ou des propriétés de matériau, telles que l'épaisseur, le poids surfacique, la résistance à la traction, l'extensibilité, la résistance à la déchirure, la drapabilité, l'activité de respiration, la capacité d'absorption, la couleur ou la transparence.

5. Champ opératoire à usage unique (1) selon l'une quelconque des revendications 1 à 4, **caractérisé en ce qu'**une deuxième section de matériau de recouvrement (18) est fixée à plat le long de la ligne de coupe (28) et double ainsi la ligne de coupe (28) sur le premier et/ou le deuxième côté supérieur de l'élément toile de base pour la formation d'un deuxième composite de couches (32), et le deuxième composite de couches (32) peut être déchiré le long de la ligne de coupe (28) sans que la deuxième section de matériau de recouvrement (18) ne se détache de l'élément toile de base.

6. Champ opératoire à usage unique (1) selon la revendication 5, **caractérisé en ce que** la deuxième section de matériau de recouvrement (18) ne comprend pas de ligne d'affaiblissement.

7. Champ opératoire à usage unique (1) selon la revendication 5 ou 6, **caractérisé en ce que** la première et la deuxième section de matériau de recouvrement (16, 18) sont formées d'une section de matériau unique.

8. Champ opératoire à usage unique (1) selon l'une quelconque des revendications 1 à 7, **caractérisé en ce que** la première et/ou la deuxième section de matériau de recouvrement (16, 18) sont fixées au moyen d'agents d'assemblage (34) extraits du groupe constitué par les agents adhésifs, tels que notamment une bande adhésive double face ou un revêtement de colle de contact, les points de soudage, tels que notamment la thermo-stratification, ou la thermo-fixation sur l'élément toile de base.

9. Champ opératoire à usage unique (1) selon l'une quelconque des revendications 1 à 8, **caractérisé en ce que** la première et/ou la deuxième section de matériau de recouvrement (16, 18) sont constituées d'un matériau polymère, notamment d'un matériau polymère extrait du groupe constitué par le polyéthylène, le polypropylène, le polyuréthane ou des mélanges de ceux-ci.

10. Champ opératoire à usage unique (1) selon la revendication 9, **caractérisé en ce que** la première et la deuxième section de matériau de recouvrement (16, 18) sont constituées du même matériau polymère.

**11.** Champ opératoire à usage unique (1) selon l'une quelconque des revendications 1 à 10, **caractérisé en ce qu'**au moins le côté supérieur (4, 6) de l'élément toile de base tourné vers la section de matériau de recouvrement (16, 18) à l'intérieur du premier et/ou du deuxième composite de couches (30, 32), notamment le premier côté supérieur (4) de l'élément toile de base, comprend un matériau en film, notamment **en ce que** le premier côté supérieur entier (4) est formé d'un matériau en film.

**12.** Champ opératoire à usage unique (1) selon l'une quelconque des revendications 1 à 11, **caractérisé en ce que** le premier côté supérieur (4) est formé d'un autre matériau ou d'un autre composite de matériaux que le deuxième côté supérieur (6), et **en ce qu'**un des côtés supérieurs (4, 6), notamment le deuxième côté supérieur (6) de l'élément toile de base, comprend un matériau absorbant, notamment un matériau non-tissé, notamment un matériau non-tissé hydrophile, ou en est formé.

**13.** Champ opératoire à usage unique (1) selon l'une quelconque des revendications 1 à 12, **caractérisé en ce que** la première zone (24) comprend un stratifié non-tissé-film, notamment en est formé, et la deuxième zone (26) comprend un film notamment transparent, notamment en est formée.

**14.** Champ opératoire à usage unique (1) selon l'une quelconque des revendications 1 à 13, **caractérisé en ce qu'**une première et/ou deuxième section de matériau de recouvrement supplémentaire (16', 18') est fixée sur un côté supérieur de l'élément toile de base le long de la ligne d'affaiblissement (14) et/ou de la ligne de coupe (28) et double ainsi la ligne d'affaiblissement et/ou la ligne de coupe, de telle sorte que la ligne d'affaiblissement et/ou la ligne de coupe est doublée par une section de matériau de recouvrement (16, 18, 16', 18') aussi bien sur le premier que sur le deuxième côté supérieur.

**15.** Champ opératoire à usage unique (1) selon l'une quelconque des revendications 1 à 14, **caractérisé en ce que** la première et/ou la deuxième section de matériau de recouvrement (16, 18, 16', 18') sont formées d'un matériau plat polymère, un côté supérieur du matériau plat comprenant un revêtement de colle de contact.

Fig. 1

Fig. 2

28

18

14

16

22

8

10

1

24

26

2

6

4

**Fig. 3**

30

16

34

4

2

6

14

**Fig. 4**

32

18

34

4

24

2

6

28

26

**Fig. 5**

Fig. 6

Fig. 7

Fig. 8

EP 3 027 136 B1

Fig. 9

Fig. 10

23

Fig. 11

Fig. 12a

Fig. 12b

Fig. 13

**EP 3 027 136 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1993462 B1
- EP 2151211 A1
- WO 2011123506 A1
- JP 2008154902 A